# EUROPEAN PATENT SPECIFICATION

(11) **EP 3 057 982 B1**
(45) Date of publication and mention of the grant of the patent: **01.11.2017**
(21) Application number: 14796268.2
(22) Date of filing: 01.10.2014
(51) Int. Cl.: C07K 14/555, C07K 14/56, C07K 14/715, A61K 38/21

(54) **FUSED PROTEINS OF INTERFERON ALPHA 5 WITH ANOTHER CYTOKINE AND A PROCESS FOR PRODUCING THEREOF**
FUSIONSPROTEINE VON INTERFERON ALPHA 5 MIT EINEM WEITEREN ZYTOKIN SOWIE VERFAHREN ZUR HERSTELLUNG DAVON
PROTÉINES D'INTERFÉRON ALPHA 5 FUSIONNÉES AVEC UNE AUTRE CYTOKINE ET LEUR PROCÉDÉ DE PRODUCTION

(30) Priority: 15.10.2013 LT 2013118
(43) Date of publication of application: 24.08.2016
(73) Proprietor: UAB Biotechnologines Farmacijos Centras "Biotechpharma", 08412 Vilnius (LT)
(72) Inventor: BUMELIS, Vladas Algirdas, LT-10103 Vilnius (LT); SUDZIUVIENE, Saule, LT-09118 Vilnius (LT)
(74) Representative: Gerasimovic, Liudmila
(86) International application number: PCT/IB2014/065004
(87) International publication number: WO 2015/056125

(56) References cited:
- WO-A1-2009/019456
- WO-A2-2011/064758
- SAULE SUDZIUVIENE ET AL: "Interferon alpha 5 and human serum albumin fused protein: strategies for cloning and display of genetic instability", BIOLOGIJA, vol. 58, no. 4, 1 January 2012 (2012-01-01), pages 233-243, XP055167752, ISSN: 1392-0146, DOI: 10.6001/biologija.v58i4.2599
- RADHAKRISHNAN R ET AL: "Zinc Mediated Dimer of Human Interferon-a2b Revealed by X-Ray Crystallography", STRUCTURE, CURRENT BIOLOGY LTD., PHILADELPHIA, PA, US, vol. 4, no. 12, 1 December 1996 (1996-12-01), pages 1453-1463, XP003013146, ISSN: 0969-2126, DOI: 10.1016/S0969-2126(96)00152-9

## Description

### FIELD OF THE INVENTION

The present invention relates to an immunomodulatory chimeric protein useful as antiviral and antitumor agent. Preferably, the present invention relates to a gene, encoding human interferon alpha, fused with the same or another interferon alpha (preferably human interferon alpha 2b) or cytokine (preferably human native mature oncostatin M protein). Chimeric fusion may include IFNa5 as N-terminal or C-terminal member of the construct. The present invention also relates to inserting the said chimeric gene in a suitable host; producing the culture of recombinant strain and stimulating expression of the heterologous polypeptide and its accumulation into insoluble inclusion bodies. The invention also provides a method for fermentation process for production of above mentioned fused protein along with a suitable protein purification process for the same. Particularly, this invention relates to the preparation of fused protein using a corresponding gene inserted in a recombinant Escherichia coli strain, thus providing the N-methionylated, mature form of protein. The invention also relates to a process for isolating and purifying said protein from insoluble fraction of bacterial strain during solubilizing, oxidizing and subjecting the material to chromatography.

The preparation of recovered protein is formulated for stability.

### BACKGROUND OF THE INVENTION

Interferons, as prominent participants in most cancer and hepatitis diseases' treatments, are important proteins in pharmaceutical market. These cytokines have antiviral, antitumor and immunomodulating activities and are widely expressed during various immunogenic processes. IFNa2b and IFNa5 are alpha interferons which bind to the same IFNAR1/2 receptor complex with different affinities and can complement each other, e.g. in hepatite C treatment. The use of OSM in malignancies is commonly associated with neutropenic infections during therapy, such as breast cancer, lymphoma, and leukemia.

Interferons have antiviral, antitumor and immunomodulating activities, as well as the effect on cell growth and differentiation with cross-activity on hormonal (epinefrin, adrenocorticotropin) functions (Dummer, R.; Mongana, J. 2009. Long term pegylated inteferon-α and its potential in the treatment of melanoma. Biologics: targets & therapy 3: 169 - 182). Currently, interferons can be regarded as important group of biopharmaceuticals in various autoimmune and cancerous diseases treatments, as well as preparates stimulating immune response to viral pathogens. Recombinant preparations of interferons are registered in the market for use in treating several cancer varieties (alpha interferons, as well as natural mixture of plasma interferons), hepatitis B and C (alpha interferons), multiple sclerosis (beta interferon), rheumatoid arthritis (gamma interferons) and as adjuvants or co-adjuvants in prophylactic vaccines (Crommelin, D. J. A.; Sindelar, R. D. 1997. Pharmaceutical biotechnology. Harwood academic publishers. 369 p. ISBN 90-5702-249-4; Wang, H., et al. 2010. Recombinant human interferon-like proteins. US patent No: 20100099612. Wang, W., et al. 2008. Effects of high-dose IFNa2b on regional lymph node metastases of human melanoma: modulation of STAT5, FOXP3, and IL-7. Clinical cancer research 14: 8314 - 8320). Interferon alpha has also been used for the treatment hairy cell leukemia, AIDS-related Kaposi's sarcoma, chronic myelogenous leukemia, and renal cell carcinoma.

With their antiviral and antitumor activities, accompanied by their influence on innate nonspecific immunity reactions, most medicinal therapies use alpha-interferons as recombinant proteins or natural mixtures secreted by infected human lymphocyte cell cultures.

Plenty of patents describe various recombinant interferon preparations in several host organisms (WO2004039996; WO 2007022799; WO 2011109556, etc).

However, these protein preparations are mostly short lived in organism, which results in high concentration doses in therapeutic uses, followed by strong adverse side effects (Crommelin, D. J. A.; Sindelar, R. D. 1997. Pharmaceutical biotechnology. Harwood academic publishers. 369 p. ISBN 90-5702-249-4). In order to reduce these effects, chimeric analogues of interferons are constructed with lower toxicity levels and longer half-life terms in plasma; thus, the concentration of dose can be lowered (WO2007002233).

To improve the pharmacokinetics of interferons and decrease dosage frequency, several stabilizing strategies have been developed, such as association with chemical species such as 2-sulfo-9-fluorenymethoxycarbonyl, recombinant long-acting fusion protein of human albumin, or other approaches such as linking the protein to larger structures such as polyethylene glycol.

Interferon alpha 2b is one of the most broadly investigated subtypes from above-mentioned family, dedicated for treatments of different varieties of cancer and cases of hepatitis B and C (Crommelin, D. J. A.; Sindelar, R. D. 1997. Pharmaceutical biotechnology. Harwood academic publishers. 369 p. ISBN 90-5702-249-4). It has been shown (WO9958143) that interferon alpha 5 protein has a great therapeutic potential on hepatitis C case due to decreased levels of this protein during this infection.

Different recombinant analogues of these proteins are known to be constructed - fusions with human serum albumin, various alpha interferon fragments or segments of Ig Fc receptor (WO0069913), polypeptide and polynuocleotide combination fragments for therapies and diagnostics (WO03000896). Great deal of research is thrown on combination therapies of IFNa2b and IFNa5 - yet it remains to be elucidated whether the therapy of both proteins results in greater efficacy of preparation.

Most interferons, among them aforementioned IFNa2b and IFNa5 are expressed in recombinant bacterial systems due to their high productivity and non-glycosylated nature of proteins (Loignon, M., et al. 2008. Stable high volumetric production if glycosylated human recombinant IFNalpha2b in HEK293 cells. BMC Biotechnology 8:65).

Both IFNa2b and IFNa5 bind to the same IFNAR1/2 receptor complex, yet launch slightly different cascades (Jaks E, Gavutis M, Uzé G, Martal J, Piehler J: Differential receptor subunit affinities of type I interferons govern differential signal activation. J Mol Biol 2007, 366(2):525-539.). Though cytokine co-vaccination for development of effective viral vaccines has shown promising potential and specifically has improved immunity afforded by viral DNA vaccines for HIV, still, combinatorial therapy for IFNa5 and IFNa6 on transgene mice has not been significant enough in clinical disease score.

A complex in a form of a fusion protein, where a type I IFN is bound to human interferon α/β receptor by covalent bond or peptide linker is disclosed in EP1037658 (prior. 19.12.1997).

This fusion imitates a certain metabolic situation when type I interferons circulate in organism binded with their soluble receptors. When certain concentration of IFN and its receptor is reached, it can reduce and slow down the immune response due to competitive binding of soluble receptors in blood plasma. Therefore, such fusion protein can exhibit both pro- and anti-antiflamatory responses depending on patient and course of disease. EP2412730 (prior. 27.03.2009) discloses a fusion protein, comprising interferon-alpha protein fused to a cytoplasmic transduction peptide and further comprises polyethylene glycol bound thereto. It is allowing to develop protein-based medicines effective for preventing or treating various liver diseases, associated with viral infection, at low doses.

Since introduction of PEGylation technologies in drug substance preparation, this became one of the most important strategies when prolongating pharmacokinetic activity. However, PEGylation strongly inhibits cellular uptake and endosomal escape, which results in significant loss of activity of the delivery system. The large PEG groups increase the hydrodynamic volume of the molecule such that it is less likely to be excreted by the kidney due to low levels of permeability through the renal basement membrane. These two features of pegylated protein can be detrimental in long run clinical trials even though they have a prolonged and low-dose action mode.

A fusion protein having the biological activity of interferon-alpha and being able to concentrate interferon-alpha in the liver is known from the patent EP1187852 (prior. 19.05.1999). Such fusion protein comprises in N-to C-terminal direction an immunoglobulin Fc region deriving from IgG1 or IgG3 and a target protein comprising interferon-alpha, where Fc region and target protein are linked together directly or by means of polypeptide linker. The fused protein is used for the treatment of liver diseases, especially hepatitis. Above mentioned fusion protein has two main disadvantages. Said fusion protein is directed to liver and used only for hepatitis B and C treatment. Incorporation of above mentioned immunoglobulin Fc regions can lead to formation of neutralizing antibodies in prolonged therapies resulting in lower therapeutic effect and patient's resistance to long treatment. However, this patent's fused protein is more versatile - when IFN alpha 5 is fused to interferon alpha2b, it can be directed for recurring melanoma treatment. Alpha 2b fusions (both IFNa5-IFNa2b and IFNa2b-IFNa5) can be directed to immunity modulation and various cancer (melanomas, lymphomas, and sarcomas) treatments, as well as viruses such as Middle East respiratory syndrome coronavirus, or skin conditions like skin/mucosal lesions caused by HPV infections. When fused with cytokine OSM, this protein can be directed to chronic hepatitis C treatment, when single IFNa5 therapy fails. Overall, our fusion proteins do not target one particular human organ, exhibit features of combination therapy and can be directed to treat broad range of viral and cancer diseases.

It is clear from the above, that the need to have a slower plasma clearance rate, thus leading to lower doses and reduced side-effects is real and actual problem in the art. Thus, creating a fused protein made from two full length interferons would allow to test this chimeric protein for its antiviral activity and clearing rate.
Another serious technical problem is related with difficulties in isolation and especially in purification of such new fused proteins. To overcome such problem the sustainable system of whole process for effective obtaining of target product and preserving where possible the activity type of the fusion partners should be invented.

### Definitions

For the sake of clarity the meaning of some terms and expressions as used in the context of the present invention is hereby provided.

As used hereinafter the term "interferon alpha 5" (or IFNa5) refers to a protein produced by leukocytes which apparently is mainly involved in innate immune response against viral infection, capable of binding to a specific cell surface receptor complex known as the IFNa receptor (IFNAR). IFNa5 includes proteins having (i) an amino acid sequence that is at least substantially identical to the amino acid sequence of a native IFNa5 protein and (II) a biological activity that is common to native IFNa5.

The term "interferon alpha 2b" (IFNa2b) refers to a produced by normal human leukocytes is predominantly interferon-alpha 2b. IFNa2b produced by leukocytes is a member of interferon family and is mainly involved in innate immune response against a broad range of viral infections, such as hepatitis B and C. Patients with recurrent melanomas receive recombinant IFN-a2b, as well as to function in an immunomodulatory fashion to promote an innate immune system response to viral pathogens by acting upon external cellular receptors and stimulating a host of intracellular and extracellular activities.

The term "oncostatin M" (OSM) refers to a protein produced by activated T lymphocytes, monocytes, microglia. It is structurally and functionally related to the subfamily of hematopoietic and neutrophic cytokines known as the interleukin 6 (IL6)-type cytokine family. Human OSM signaling is mediated by its binding to two receptor complexes: the type I OSM receptor complex (LIF receptor) consisting of interleukin 6 signal transducer (gp130) and Leukemia inhibitory factor receptor subunits (LIFR), and the type II OSM complex (OSM receptor) consisting of gp130 and OSM receptor beta (OSMR) subunits.

As used herein the term "chimeric IFNa5 protein" refers to a protein that is produced by a microorganism that has been transformed with a fused gene, encoding a protein having an amino acid sequence, that is at least substantially identical to the amino acid sequence of native human IFNa5 as either N-terminal or C-terminal member.

"Substantially identical amino acid sequence" means that the sequences are identical or differ by one or more (up to 5%) conservative amino acid mutation or alteration (i.e. deletions, additions, substitutions) that do not produce an adverse functional dissimilarity between the recombinant protein and native human IFNa5, IFNa2b or OSM. The percentage of sequence identity may be obtained by using the BLAST program (blast2seq, default parameters) [Tatutsova and Madden, FEMS Microbiol. Lett., 174, 247-250 (1990)].

As used herein the term "microorganism, producing IFNa5-fused protein" refers to a microorganism that has been genetically engineered to produce a protein that possesses biological activity associated with human IFNa5. Correspondingly the term "E.coli host cell, producing IFNa5 fused protein" refers to an E.coli host cell that has been genetically engineered to produce a protein, possessing biological activity associated with an IFNa5. The terms "plasmid", "vector system" or "expression vector" means a construct capable of in vivo or in vitro expression. In the context of the present invention, these constructs may be used to introduce genes encoding target fused proteins into host cells.

Preferably, the expression vector is incorporated into the genome of a suitable host organism. The term "incorporated" preferably covers stable incorporation into the genome. The term "regulatory sequences" includes promoters and enhancers and other expression regulation signals. A regulatory sequence "operably linked" to a coding sequence is ligated in such a way that expression of the coding sequence is achieved under condition compatible with the control sequences.

The term "promoter" is used in the normal sense of the art, e.g. an RNA polymerase binding site.

The term "host cell" - in relation to the present invention includes any cell that comprises either the nucleotide sequence or an expression vector as described above and which is used in the recombinant production of a target fused protein having the specific properties as defined herein or in the methods of the present invention.

By "isolated" and/or "purified" is meant that the IFNa5-fused protein is in a relatively pure state - e.g. at least about 90% pure, preferably at least about 95% pure, most preferably at least about 98% pure.

### DISCLOSURE OF THE INTENTION

### Summary of the Invention

The principal object of the present invention is fused protein, comprising an interferon alpha 5 (IFNa5), of a general formula (I) or (II)

IFNa5 - X (I)

X - IFNa5 (II)

wherein fusion partner X is another alpha interferon or cytokine.

More particularly, the present invention covers fused proteins, wherein IFNa5 is human recombinant interferon alpha 5 and X is selected from the group, consisting of IFNa5, INFa2b and oncostatin. The objects of present invention cover such proteins as:
IFNa5-IFNa5 homodimer with amino acid sequence of SEQ ID No: 1 or at leat 95% identical sequence;
IFNa2b-IFNa5 heterodimer with amino acid sequence of SEQ ID No: 2 or at least 95% identical sequence;
IFNa5-IFNa2b heterodimer with amino acid sequence of SEQ ID No: 3 or at least 95% identical sequence;
IFNa5-OSM heterodimer with amino acid sequence is SEQ ID No: 4 or at least 95% identical sequence;
OSM-IFNa5 heterodimer, which amino acid sequence is SEQ ID No: 5 or at least 95% identical sequence.

In the preferred embodiment fused protein of present invention is exhibiting a biological actyvity, characteristic to IFNa5, along with prolonged clearance rate in rat serum samples. DNA fragment, encoding said fused protein, has a coding sequence, selected from the group, consisting of SEQ ID NO: 6, SEQ ID NO: 7, SEQ ID NO: 8, SEQ ID NO: 9, SEQ ID NO: 10 or is the sequence, coding the same protein. A suitable vector, comprising DNA fragment, encoding fused protein according to present invention, is selected from the group, consisting of pET21b+, pET28a+, pUC57/T and pT7, preferably is pET21b+.

Another aspect of present invention is a process for producing a fused protein of the invention by expression in an appropriate host cell, comprising:
a) providing of host cell, producing said IFNa5 fused protein;
b) cultivating host cells under conditions effective to express said IFNa5 fused protein in a suitable fermentation medium;
c) isolating and purifying the expressed IFNa5 fused protein.

The process according to invention is characterised in that:
- said isolation is isolation from the obtained in step b) mixture, comprising the target fused protein in a form of inclusion bodies, by subjecting them to solubilization and following oxidizing renaturation; and
- said purification is 3-step chromatographic treatment comprising:
   subjecting said mixture comprising renatured IFNa5 fused protein to a hydrophobic interaction chromatography;
   following by subjecting the solution obtained in the previous step to an anion-exchange chromatography;
   and further subjecting the solution obtained in the previous step to a cation-exchange chromatography.

The conditions effective to express said IFN fused protein comprise induction, preferably with isopropyl-β-D-thiogalactopyranoside (IPTG) at concentration of 0.1mM to 1mM, most preferably 0.5 mM, and induction is optionally shifted to early growth stage.

In one of embodiments the host cell is selected from the group, consisting of E.coli, S.cerevisiae, K.lactis, preferably E.coli, more preferably E.coli protease deficient strain, such as E.coli BL21 and Rosetta gami-2, most preferably an E.coli BL21, and wherein suitable fermentation medium is any complex medium containing yeast extract as nitrogen source and glucose as carbon source, preferably M9m medium.

In the other embodiment the solubilization step is performed in chaotropic and denaturing solution, comprising of 6-8 M urea or 4-6 M guanidine hydrochloride, preferably 6 M guanidine hydrochloride, optionally with 20 mM DTT.
The oxidising renaturation according to the preferred embodiment of present invention is performed in glycine/NaOH buffer at pH in the range of 7,0 - 9,6 and pair of reduced/oxidised glutathione GSH/GSSG in the ratio 5-20:1 with buffering additives, selected from the group, comprising sodium chloride, sodium sulphate and ammonium sulphate.
In said 3-step chromatographic purification according to present invention the hydrophobic interaction chromatography sorbent is selected from the group, consisting of Butyl-Sepharose, Octyl-Sepharose, Phenyl-Sepharose, preferably Phenyl-Sepharose in pH range of 8,2-9,2; the anion-exchange chromatography sorbent is selected from the group, consisting of ANX Sepharose, Q-Sepharose, DEAE-Sepharose, QAE-Sepharose, preferably Q-Sepharose or DEAE-Sepharose in pH range of 8,6-9,0; and the cation-exchange chromatography sorbent is selected from the group, consisting of S-Sepharose, SP-Sepharose and CM-Sepharose, preferably SP-Sepharose or CM-Sepharose in pH range of 5,2-5,6.

The further aspects of present invention are fused protein, obtained by the process of invention and preparation, comprising the fused protein according to present invention in the form of stable solution. Both fused protein and preparation according to present invention are for use in therapy.

Pharmaceutical composition according to the invention is comprising therapeutically effective amount of said fused protein or said preparation and pharmaceutically acceptable carrier, excipient and/or auxiliary substances.

The invention is described below in further details and with reference to the accompanying drawings and examples.

### BRIEF DESCRIPTION OF THE DRAWINGS:

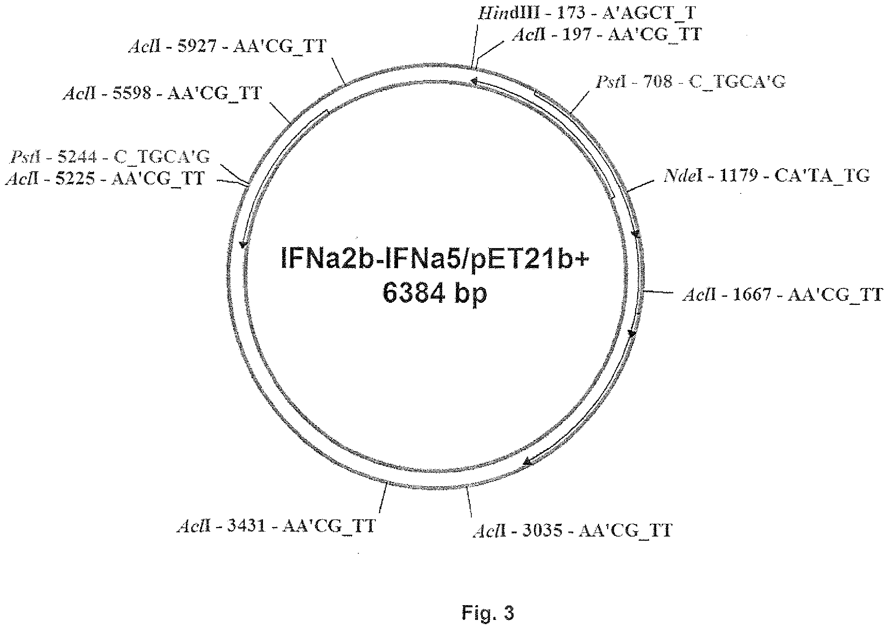

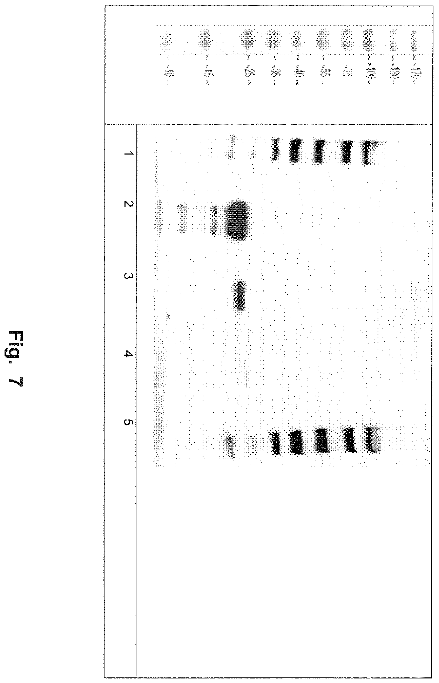

The preparation of fused IFNa5 proteins according to present invention is illustrated by Figures 1-9.
Fig. 1 is cloning scheme of IFNa5-IFNa5 homodimer.
Fig.2 shows cloning scheme of IFNa2b-IFNa5 heterodimer.
Fig. 3 is map of IFNa2b-IFNa5/pET21b+ with restriction endonucleases used for restriction pattern analysis of clones.
Fig. 4 represents growth curves and biosynthesis parameter characteristics in different growth media on example of IFNa2b-IFNa5/pET21b+/E.cali BL21(DE3) strain.
Fig. 5 represents an image of SDS-PAGE and Western-blot analysis of IFNa2b-IFN a5 expressed in E.coli BL21(DE3)/pET21b+. Lane 1 - molecular weight protein marker; lane 2 - total protein fraction (before induction); lane 3 - total protein fraction (2.5 h after induction); lane 4 -soluble protein fraction (2.5 h after induction); lane 5 - insoluble protein fraction (2.5 h after induction); lane 6 - IFNa5 reference solution.
Fig. 6 represents SE-HPLC of IFNa2b-IFNa5 purified protein.
Fig. 7 illustrates patterns of Silver-stained 15% SDS-PAGE under reduced conditions for formulated IFNa2b-IFNa5 fusion substance. Lane 2 - sample from SP-Sepharose FF column, fr. A6 10 µg, lane 3 - sample from SP-Sepharose FF column, fr. A6 1 µg, lane 4 - sample from SP-Sepharose FF column, fr. A6 0,1 µg. Lanes 1 and 5 - Protein Molecular Weight Marker (Fermentas, #26616, lot. #00079685).
Fig. 8 is HPLC view of IFNa5-IFNa2b peptide mapping.

### Detailed Description of the Invention

### Objects of the invention

The present invention aims to provide a method of producing recombinant DNA coding for a IFNa5-fused protein which displays physicochemical properties similar to mature native IFNa5. Another objective of the present invention is to produce either of IFNa5-fused proteins by controlled fermentation. Yet another objective of the present invention is to obtain IFNa5-fused protein in a pure form. A further object of the present invention is to prepare a pharmaceutical composition, comprising of the said IFNa5-fused protein.

As mentioned above the main objects of present invention are fused IFNa5 proteins of general formulas (I) or (II), obtainable by the process of present invention, where both partners are fused directly, without the linker. Additional linker amino acid sequences are not always desirable for their unpredicted toxicity, influence on folding and proteolysis processes and biosimilarity issues.
In particular embodiments of invention the suitable fused IFNa5 proteins are those listed in the table 1 below.

**Table 1**

| Sequence ID NO: | Fused protein | Molecular weight | Amino acid sequence |
|---|---|---|---|
| 1 | IFNa5-IFNa5 | 39161.7 | |
| 2 | IFNa2b-IFNa5 | 38906.5 | |
| 3 | IFNa5-IFNa2b | 38906.5 | |
| | | | |
| 4 | IFNa5-OSM | 41788.7 | |
| 5 | OSM-IFNa5 | 41788.7 | |

A microorganism producing IFNa5-fused protein can be generated by introducing a DNA fragment /nucleic acid molecule, encoding a chimeric protein.
In particular embodiments of invention the suitable sequences encoding the fused proteins of IFNa5 of present invention are ones listed in the Table 2 below.

**Table 2**

| SEQ ID NO: | Fused protein | Encoding sequence |
|---|---|---|
| 6 | IFNa5-IFNa5 | |
| | | |
| 8 | IFNa5-IFNa2b | |
| 7 | IFNa2b-IFNa5 | |
| | | |
| 9 | IFNa5-OSM | |
| 10 | OSM-IFNa5 | |
| | | |

Suitably the coding sequence may be part of an expression vector. The nucleotide sequences described herein including the nucleotide sequence of the present invention may be present in a vector in which the nucleotide sequence is operably linked to regulatory sequences capable of providing for the expression of the nucleotide sequence by a suitable host organism.
The vectors for use in the present invention may be transformed into a suitable host cell as described below to provide for expression of a fused polypeptide of the present invention.

The choice of vector e.g. a plasmid, cosmid, or phage vector will often depend on the host cell into which it is to be introduced. Suitable vectors include pET21b+, pET28a+, pUC57/T and pT7, pET21b+ being preferred.
The vectors for use in the present invention may contain one or more selectable marker genes such as a gene, which confers antibiotic resistance e.g. ampicillin, kanamycin, chloramphenicol or tetracycline resistance. Alternatively, the selection may be accomplished by co-transformation.
Vectors may be used in vitro, for example for the production of RNA or used to transfect, transform, transduce or infect a host cell.
Thus, in a further embodiment, the invention provides a method of making nucleotide sequences of the present invention by introducing a nucleotide sequence (DNA fragment) of the present invention into a replicable vector, introducing the vector into a compatible host cell, and growing the host cell under conditions which bring about replication of the vector.
The vector may further comprise a nucleotide sequence enabling the vector to replicate in the host cell in question. Examples of such sequences are the origins of replication of plasmids pUC19, pACYC177, pUBHO, pE194, pAMBI, pIJ702 and pBR322.

Enhanced expression of the nucleotide sequence encoding IFNa5-fused protein may also be achieved by the selection of heterologous regulatory regions, e.g. promoter, secretion leader and terminator regions.
Preferably, the nucleotide sequence according to the present invention is operably linked to at least a promoter. Examples of suitable promoters for directing the transcription of the nucleotide sequence in suitable bacterial host cells are well known in the art.
The embodiments of the present invention provide host cells transformed or transfected with a nucleotide sequence that expresses chimeric protein as described herein. The cells will be chosen to be compatible with the said vector and may for example be prokaryotic (for example bacterial), fungal, yeast or plant cells. Preferably, the host cells are prokaryotic cells.
Examples of suitable bacterial host organisms are gram positive or gram negative bacterial species.
Suitable bacterial host cells include Escherichia coli. Suitably E. coli strains include BL21, BL21(DE3), Rosetta gami-2, JMI09, K12 and K802, preferably BL21(DE3) and Rosetta gami-2.

The genotype of the host cell may be modified to improve expression. Examples of host cell modifications include protease deficiency, supplementation of rare tRNA's, and modification of the reductive potential in the cytoplasm to enhance disulphide bond formation, such as thioredoxin reductase (trxB) and gluthation oxidoreductase (gor) mutations.
For example, the host cell E. coli may over-express rare tRNA's to improve expression of heterologous proteins as exemplified/described in Kane [Curr Opin Biotechnol (1995), 6, 494-500 "Effects of rare codon clusters on high-level expression of heterologous proteins in E. coli"]. The host cell may be deficient in a number of reducing enzymes thus favouring formation of stable disulphide bonds as exemplified/described in Bessette [Proc Natl Acad Sci USA (1999), 96, 13703-13708 "Efficient folding of proteins with multiple disulphide bonds in the Escherichia coli cytoplasm"].
In particular embodiment, the E.coli host cell is an E.coli protease deficient strain, such as E. coli lon/omp T-protease deficient host strain, preferably an E. coli BL21 strain, most preferably an E.coly BL21 (DE3) strain.
The transformed E.coli host of present invention contained an expression vector prepared from pET21b+ (Novagene).

According to the present invention said fused gene is cloned into pET21b+ vector downstream to early bacteriophage T7 promoter. The gene is said to be in a reading frame from promoter, lac operon should be upstream from T7 promoter, and the expression of desired fused construct, containing open reading frame, is stringently controlled and terminated by T7 terminator.

In the main aspect the invention related to a process for producing an IFNa5 fused protein by expression in an IFNa5 producing E.coli host cell, which comprises:
a) providing an E.coli host cell, producing said IFNa5 fused protein;
b) culturing E. coli host cells producing IFNa5 fused protein under conditions effective to express said IFNa5 fused protein by said recombinant E.coli host cell in a suitable fermentation medium, wherein said fermentation medium is comprised of components from animal origin or yeast origin;
c) isolating and purifying the expressed IFNa5 fused protein.

In particular embodiment, said recombinant IFNa5 fused protein which may be produced according to the process of the invention is an IFNa5 fused protein of which one of the partners is substantially identical to the native IFNa5, i.e., a protein that is produced by E.coli host cell that has been transformed with gene, encoding chimeric IFNa5 fused protein, or a modification thereof, that encodes a protein, having (1) amino acid sequence that is at least substantially identical to the amino acid sequence of native IFNa5 fused with any other cytokine and (2) a biological activity that is common to native IFNa5, In a preferred embodiment, said IFNa5 is hIFNa5.
In a more particular embodiment, the recombinant IFNa5 fused protein produced according to the invention is a chimeric protein having either amino acid sequence shown in SEQ ID NO: 6, SEQ ID NO: 7, SEQ ID NO: 8, SEQ ID NO: 9 or SEQ ID NO: 10 (Table 2 above).

The E. coli host cell, producing IFNa5 fused protein, can be obtained by conventional methods and protocols for cloning and expressing [e.g., Sambrook et al. Molecular cloning: A Laboratory Manual. Second ed., CSH Laboratory, Cold Spring Harbor, 1989; Current Protocols in Molecular Biology, vol. 1-3 (Ausubel F.M. et al., ed.) John Wiley & Sons, Inc., Brooklyn, New York, 1994-1998].
In particular embodiment, the cloning and expression of an IFNa5 encoding gene and construction of the bacterial strain producing recombinant IFNa5 protein (i.e., the E. coli host cell producing the IFNa5) can be performed according to a method which comprises cloning of the cDNA gene encoding chimeric gene, modification of DNA sequence of said gene to optimize its expression in E. coli, construction of the expression plasmid, transformation of the selected plasmid into suitable E. coli strain and selection of the expression/induction conditions. Examples 1, 2, 3 and 4 disclose the details of construction of E.coli host cell, producing chimeric hIFNa5-fused protein.

In a particular embodiment, when the E.coli host cell is an E. coli protease deficient strain, such as E. coli BL21 (DE3) strain, said host cell is transformed with a vector, comprising a sequence encoding the IFNa5 fused protein under the control of an inducible promoter. In that case the expression of the protein, as mentioned above, requires the addition of an inducer, such as, for example, isopropyl-β-D-thiogalactopyranoside (IPTG). Thus, according to a particular embodiment, when the E. coli host cell is an E. coli BL21 strain, such as an E. coli BL21 (DE3) strain, the conditions of step b) comprise induction. The expression of recombinant IFNa5-fused protein is induced after reaching appropriate biomass buildup using suitable lac operon inducer, such as lactose and the like, preferably IPTG at concentration of 0.1mM to 1mM, most preferably 0.5 mM.

The culture medium of step b) of the process of the invention is selected from complex media such as LB, YPD, TB, M9 or M9m, wherein said culture medium comprises one or more nitrogen sources, selected from the group consisting of ammonium salts, nitrates, tryptone, yeast extract and ammonium hydroxide, and a carbon source, selected from the group consisting of glycerol, glucose, fructose and the like, preferably glucose.
The biomass build up is in a range of 10 to 50 g/L, preferably 25-40 g/L for complex medium media based on wet cell weight, wherein said culture medium has the following parameters:
- pH in the range of 3.0 to 8.0, preferably 6.0 to 6.8 for complex medium;
- temperature in the range of 25 to 45 degree C, preferably 37 to 42 degree C, and
- dissolved oxygen: 20-80% of saturation, preferably 20-40% of saturation;
and said culturing is carried out for a duration of 4 to 10 hours, preferably 6 hours.

The isolating and purifying of target IFNa5-fused protein from a protein producing microorganism, i.e. step c) of the process of present invention, are comprising:
- lysing the microorganism and separating insoluble material comprising fused protein from soluble proteinaceous cell material;
- solubilising the fused protein present in the insoluble material;
- oxidizing the fused protein in the presence of a pair oxidizing/reducing agent;
- subjecting the solution to chromatography; and
- recovering purified fused protein.

More specifically the step of isolating and purifying of IFNa5-fused protein from a producing microorganism comprises:
- lysing the microorganism and separating insoluble material containing recombinant IFNa5-fused protein from soluble proteinaceous material;
- solubilizing the recombinant protein present in the insoluble material;
- oxidizing the IFNa5-fused protein using oxidized glutathione in the presence of reduced glutathione;
- separating of refolded target protein from chaotropic agent;
- combination of suitable chromatography purification methods.

In preferred embodiment of the present invention in step c) the IFNa5 fused protein in the insoluble material is solubilized using a chaotropic agent, preferably at an intermediate concentration of a chaotropic agent, at pH in the range 8.00-9.00. The solubilising might be performed by using guanidinium hydrochloride, the concentration of guanidinium hydrochloride being from 3.0 to 7.0, preferably 6.0 - 6.2 M.
In one of preferred embodiments of the present invention the pair of oxidizing/reducing agent is a pair of oxidized/reduced glutathiones, wherein the molar ratio of oxidized and reduced glutathione is from 1:5 to 1:20, preferably 1:20.
The embodiment of the present invention further comprises separation of the refolded IFNa5 fused protein from chaotrope, wherein the refolded IFNa5 fused protein is separated from chaotrope by hydrophobic interaction chromatography.
The chromatography of step c) is preferably combination of 3-step chromatography purification, comprising hydrophobic interaction chromatography, cationic exchange chromatography and anionic exchange chromatography.

Various hydrophobic interaction chromatography supports, selected from the group, consisting of BioBeads SM-2, Butyl-S-Sepharose, Capto Octyl, Octyl Sepharose, Phenyl Sepharose, Capto Butyl, Butyl Sepharose might be used.

Various anion exchange chromatography supports, selected from the group, consisting of UnoSphere Q, Macro-Prep High Q, Macro-Prep DEAE, Macro-prep 25 Q, DEAE Sephacel, ANX Sepharose 4, Q Sepharose HP might be used.
Various cationic exchange chromatography supports can be used and may be selected from the group comprising: SP Sepharose FF, SP Sepharose HP, CM Sepharose FF, TSK gel SP-5PW, TSK gel SP-5PW-HR, Toyopearl SP-650M, Toyopearl SP-650S, Toyopearl SP-650C, Toyopearl CM-650M, Toyopearl CM-650S, Macro-Prep High S support, Macro-Prep S support, Macro-Prep CM support etc.

Stable solution of target fusion protein is formulated for use in therapy and stability studies. Pharmaceutically acceptable carriers for this fusion protein are water, saline, acetate or phosphate buffered saline, dextrose, glycerol and the like, as well as combinations thereof. In many cases, it will be preferable to include isotonic agents, for example, sugars (mannose or ramnose), polyalcohols such as mannitol, sorbitol, or small compounds such as sodium chloride or glycine in the composition. It is considered that in all cases sterile solutions are prepared: fusion molecule is incorporated in appropriate solvent after purification process and is sterile filtered.

Possible indications are related with indications of fusion partners: acute and chronic hepatitis B and C, cancerous diseases such as melanomas, lymphomas and sarcomas, broad range of viral infections (Middle East respiratory syndrome coronavirus, or skin conditions like skin/mucosal lesions caused by HPV infections) and anti-inflammatory treatments of rheumatoid arthritis.

### Description of the embodiments of the invention

As mentioned, key strategy of the present invention for fusing genes is to maintain the same amino acid sequence - without using any linkers between the genes. This is done in order to avoid certain immunogenicity of linker sequences. The experiments generally consisted of simple sequence - fusing genes by gene engineering methods, DNA transformation into the recombinant E.coli and selection of clones, biosynthesis optimization in complex medium and finally, selecting an appropriate scheme for purification of recombinant protein.

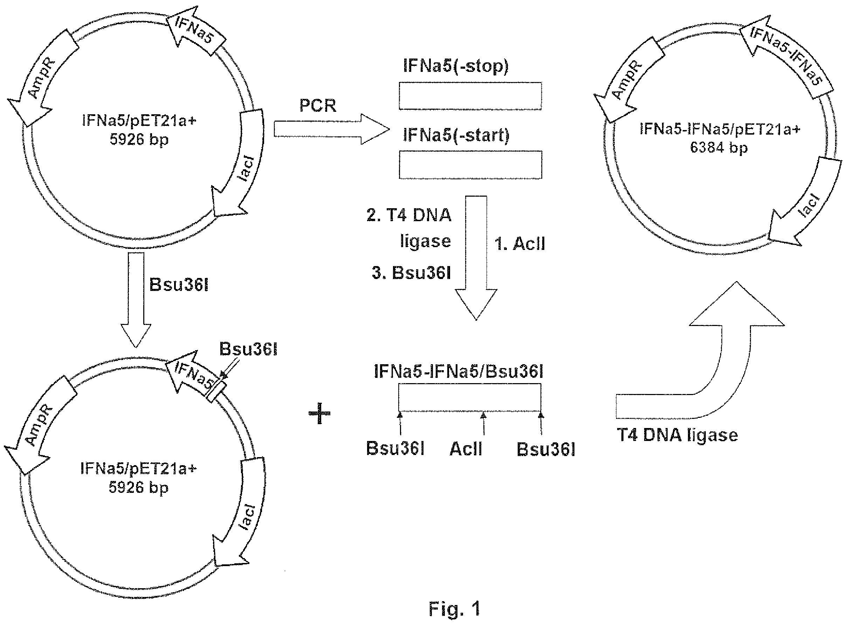

All genetic alterations were performed by introducing mutations into sequences without altering main amino acid sequence. Main strategy for the fusion cloning procedures were PCR optimization, gene fusing via sticky ends and extraction of suitable size and orientation gene product, it's ligation into transient or expression plasmid and transformation to various E.coli strains. All clones obtained were checked for their insert size, orientation, expression levels; restriction analysis and sequencing were performed on selected clones. For interferon alpha 5 dimer fusion, a specific and unique cloning sequence was performed (detailed description in Example 1 and Fig. 1).
The fused protein was introduced into several different plasmids expecting to find the producent with best qualities. The plasmids tested were pET21b+ and pET28+, both designated for strong control and high-level expression of recombinant proteins. Promising clones with proper restriction patterns and correct insert size were also transformed into two different E.coli strains for stable expression - E.coli BL21(DE3) and Rosetta gami-2. The best expression rate and plasmid stability ratio for selected clone were main criteria to proceed further with biosynthesis optimization.
Biosynthesis was performed in Biorad bioreactors with supplied air, maintained temperature and pH. Cultivation was performed in complex medium, optimizing it's composition, additives, temperature regimes, IPTG concentration and induction time. In most successful performed experiment (see Example 4), IFNa5-fused protein was expressed in the form of inclusion bodies and accumulated up to 40 % of insoluble cell protein fraction. Biosynthesis of interferon alpha 5 fused proteins can be described as batch biosynthesis in rich medium with yeast extract and glucose as main carbon source. During biosynthesis process optimization, several criteria were taken in attention. Yield of recombinant protein is the main criterion when evaluating biosynthesis process, however, low plasmid stability can be the key parameter to change biosynthesis conditions and to sacrifice protein yield. Low plasmid stability, which was observed in initial biosynthesis optimization stages, can lead to accumulation of non-producing E.coli cells that will burden purification process and lower subsequent yields. The use of E.coli Rosetta gami-2 strain enpowers to produce fused protein into soluble protein fraction of the cell. This can be desired when IFNa5 protein is fused to a cytokine with folding impediments. After biosynthesis process optimization, a technology was established for producing a recombinant protein with maximum best values of biosynthesis process parameters. Further details are described in Example 5.

Purification was performed by combination and optimization of various ion-exchange and hydrophobic interaction sorbents. Purification process is finely tuned for a specific process of fused protein purification. Protein folding process after denaturation step strongly relies on peptide fragments tendency to fold into certain forms. In the case of fusion proteins it has been observed during all purification stages and their analysis with RP-HPLC that a family of missfolded protein is forming around main peak of target protein. Initial trials for the fusion isolation from inclusion bodies and purification were performed. The main drawback raised from the possible overlapping interaction of exposed to the solvent -S-S-bonds (located within the loop) of both fusion partners which leads to the formation of unknown species (detected by RP-HPLC) which are hardly separated from the main fusion form. To avoid these forms, a folding and purification scheme was selected with lower yields of total protein, sacrificing part of the yield for retaining most of correctly folded fraction.
In all studied cases the refolding was performed by total 16x dilution of IB solution. For this purpose 56 ml of IB solution was poured into refolding buffer by gentle mixing final solution for defined duration at +4°C. Refolding buffer usually was composed of 25 mM glycine/NaOH buffer of defined pH value containing 1.2 M NaCl, 0,5 M L-Arg, and selected pair of reduced/oxidised glutathione, or oxidised glutathione to supplement residual DTT as reducing agent. Refolding conditions were optimised by studying the influence of pH: 7.0; 7,5; 8; 8,5; 9; 9,5 at molar ratio and concentration of GSH/GSSG=2,5mM/:0,25mM, and molar ratio and concentration of GSH/GSSG=2,5mM/:0,25mM; 5.0mM:0,25mM; 5.0mM:0.5mM; 10mM:1.0mM at pH value 9.6. Refolding efficiency was controlled by RP-HPLC. As optimized were selected those refolding conditions which gave the highest percent of correctly folded fusion form: molar ratio and concentration of GSH/GSSG=2,5mM/:0,25mM.
For IFNa2b-IFNa5 fusion protein, isolated inclusion bodies were reduced with DTT and refolding was performed introducing 0.5 mM of oxidized glutathione while the others additives of refolding buffer (1.2 M NaCl, 0.5 M L-Arg) remained the same. However, our attempts to produce larger amount of the fusions were not appropriate in terms of process yield. We found that the additives of ammonium sulphate added into refolding buffer instead of NaCl allow increase about twice the content of correctly folded fusion protein. With regard to this further fusion process was performed with inclusion into refolding buffer of 0.5 M ammonium sulphate. Such salt changes during refolding procedure evoque the changes of initial fusion chromatography steps since first chromatography purification step over Phenyl-Sepharose FF column has earlier been adjusted using NaCl. Phenyl-Sepharose FF column is dedicated for separation of refolded protein solution from residual guanidinium hydrochloride (GdmCl). Therefore, to operate Phenyl-Sepharose FF column, i.e. loading of refolded protein on the column equilibrated with 1.5 NaCl it was necessary to perform ammonium sulphate exchange to NaCl or to find proper conditions for this column operation using equilibration buffer containing proper selected concentration of ammonium sulphate. Testing of Phenyl-Sepharose FF column operation using various concentration of ammonium sulphate added to equilibration buffer was performed. Data showed that it is possible to perform chromatography of refolded protein on Phenyl-Sepharose FF column using 1.25 M ammonium sulphate. In such case performing renaturation step using additives of 0.5 M ammonium sulphate the subsequent step of chromatography over Phenyl-Sepharose FF column may be performed omitting Sephadex G-25 column. It is enough to add ammonium sulphate into solution of refolded protein and equilibration buffer up to its concentration of 1.25 M and perform chromatography analogously as in the presence of NaCl. For Phenyl Sepharose chromatography, column was equilibrated with 20 mM Tris HCl, 1.5 M NaCl, pH=8.2, conductivity 115,1 mS/cm. 2500 ml of IFNa2b-IFNa5 fusion protein solution after Sephadex G25 was loaded on the column with elution flow-rate 20 ml/min and eluted with 10 mM TrisHCl, pH= 9.2, conductivity 1196 µS/cm.
After Phenyl Sepharose, Q-Sepharose chromatography was performed: with equilibration with 20 mM Tris-HCl, 0,05 M NaCl, pH 8,8. Protein pool from Phenyl Sepharose was pH adjusted with 6M HCL to 8.8, conductivity 8mS/cm and loaded on column. Desorption gradient was performed as 20 mM TrisHCl, linear gradient 0,05 to 0,25 M NaCl, pH 8,8. Collected fraction was subjected to SP-Sepharose chromatography. SP-Sepharose column was equilibrated with 20 mM CH3COONa, 100 mM NaCl, pH 5,4. Sample was loaded after pH adjustment with 6 M HCl to 5.4, conductivity 9,5 mS/cm. Desorption from the column was with gradient of 20 mM CH3COONa, from 50 mM to 0,25 M NaCl, pH 5.4. After SP-Sepharose FF chromatography pooled fractions containing fusion were added to 1 part (10:1 volume) of concentrated phosphate buffer (250 mM phosphate, 150 mM NaCl, pH 7.0), pH corrected to 7.0 and the solution was concentrated using Millipore Ultracel (10MWCO) centrifugal filters. After concentration the buffer was exchanged to PBS (25 mM phosphate, 150 mM NaCl, pH 7.0). After buffer exchange total protein content was determined using Pierce 660 protein assay. Protein solution was filtered using Mustang E filter and aliquoted into vials for 1 ml (final concentration 1,005mg/ml) in sterile environment (Laminar airflow hood). Vials were frozen and kept at -80°C temperature. Samples were taken for RP-, SEC-HPLC, total protein and endotoxin determination.
For fusion proteins IFNa5-OSM and OSM-IFNa5, we have evaluated impact of ammonium sulfate on the renaturation yield of the fusion. All renaturation buffers consisted of 25 mM Glycine/NaOH buffers, containing, 0,5 M L-Arg, pH 9,6, 5 mM reduced (GSH) and 0,25 mM oxidized (GSSG) glutathiones. The increase of ammonium sulfate concentration in refolding buffer gave higher yield of renatured protein in comparison to earlier used additive of 1.2 M NaCl. Optimized scheme of IFNa5 and OSM fusion proteins is presented below: 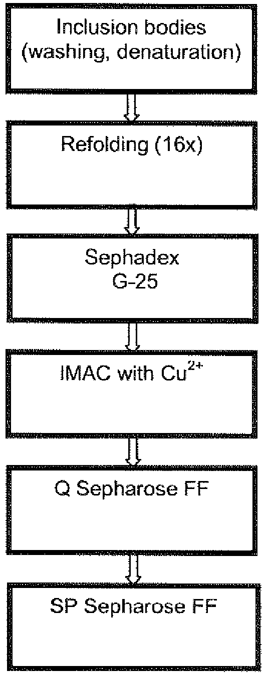 50 g of biomass was resuspended in 500 ml 0,1 M Tris-HCl buffer pH 7.4 containing 2 mM EDTA, 25 mM DTT, 250 mM PMSF, 0,1 % Triton X-100 and disrupted using ultra sonication. Disruption was carried out using impulse 1:1, amplitude 40% and time for disruption as 25 min for 10 g. Suspension obtained was cenrifuged at 12000 rpm for 30 min and the pellets containing inclusion bodies (IB) were frozen at -20°C. Washing of inclusion bodies was performed in such manner:
Twice with washing buffer I: 10 mM Tris-HCl, 1 M NaCl, 0.1 % Polysorbate-80, pH 7,4, each with 500 ml
Washing buffer II: 10 mM Tris-HCl, 6 M Urea, pH 8,0, 500 ml
Washing buffer III: 10 mM Tris-HCl, pH 8,0, 500 ml

After each washing step IB were centrifuged at 10000 rpm with Backman centrifuge.

Pre-washed IB were solubilized in 300 ml 25 mM Gly/NaOH buffer pH 8.7 containing 2 mM EDTA, 6 M GdmCl, 20 mM DTT (297 mS/cm, +4°C) for 2 hours and centrifuged thereafter at 9500 rpm for 30 min with Backman centrifuge.

Refolding was carried in Duran type 2L beakers, covered with parafilm. Solubilized inclusion bodies were 10x diluted with refolding buffer 25mM Gly/NaOH, 0,5M Arginine, 0,25M (NH4)2SO4 2,5mM GSH, 0,25mM GSSG, pH 9.6, to the filnal volume of 3000 ml. Refolding was carried approximately for 64h. After refolding protein solution was centrifuged for 30 min, 9500 rpm +4 °C in Beckman centrifuge. For Sephadex G-25, all obtained after refolding solution was loaded on column and washed with 20 mM Tris-HCl, 0.5 M NaCl, pH 9.0 for 1.2 column volumes. After desalting, protein solution was subjected to chelating sepharose chromatography. Chelating Sepharose FF media was washed with 50 mM EDTA solution, 2 CV, 0,1 M HCl solution, 2 CV, water till to pH value above 5 and charged with Cu(II) ions loading 0,2 M Cu2SO4 solution, 2 CV. The column is washed with water and equilibrated with 20 mM TrisHCl buffer, pH 7.0 containing 0,5 M NaCl at a flow rate 10 ml/min. 3250 ml desalted protein solution, pH 7.0were loaded on the column. Wash out of unbound proteins was performed with 20 mM TrisHCl, 0.5 M NaCl, pH 7.0, 2 CV. Elution was started with 20 mM TrisHCl, 0.5 M NaCl, 15 CV gradient to 100 mM imidazole, pH 7.0. Collected fractions were checked for fusion protein presence and subjected to Q-Sepharose FF media. This column was equilibrated with 20 mM TrisHCl, 70 mM NaCl, pH 9.0, 10 CV. Wash out unbound proteins: 20 mM TrisHCl, 70 mM NaCl, pH 9,0, 2 CV; and elution with 20 mM TrisHCl, 10 CV gradient to 300 mM NaCl, pH 9.0. For final stage of purification, CM Sepharose is selected. Equilibration of the column is performed with 20 mM sodium acetate, 50 mM NaCl, pH 5.5, 10 CV. Elution of the final protein with 20 mM sodium acetate, 15 CV gradient to 500 mM NaCl, pH 5.5. Pooled fractions containing fusion were added to 1 part (10:1 volume) of concentrated phosphate buffer (250 mM phosphate, 150 mM NaCl, pH 7.0), pH corrected to 7.0 and the solution was concentrated using Millipore Ultracel (10MWCO) centrifugal filters. After concentration the buffer was exchanged to PBS (25 mM phosphate, 150 mM NaCl, pH 7.0). After buffer exchange total protein content was determined using Pierce 660 protein assay. Protein solution was filtered using Mustang E filter and aliquoted into vials for 1 ml (final concentration 1,005mg/ml) in sterile environment (Laminar airflow hood). Vials were frozen and kept at - 80°C temperature. Samples were taken for RP-, SEC-HPLC, total protein and endotoxin determination.

For IFNa5-IFNa5 fusion protein, a third purification scheme was optimized.

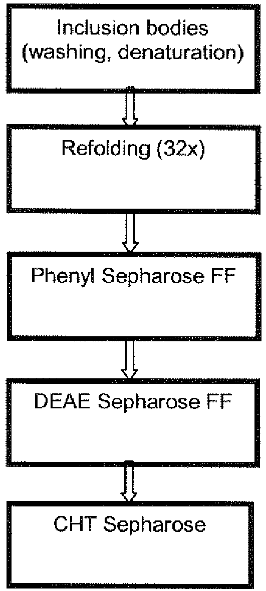

Pre-washing of isolated inclusion bodies (IB) was performed by a four-consecutive steps procedure using: washing buffer I: 10 mM Tris-HCl buffer, containing 1 M NaCl, 0,1% Polysorbate-80, pH 7,4; washing buffer II: 10 mM Tris-HCl buffer containing 6 M Urea, pH 8,0 and washing buffer III: 10 mM Tris-HCl buffer, pH 8.0.

Solubilization buffer is consisting of 25 mM Gly/NaOH buffer containing 2 mM EDTA, 6 M GdmHCl, 20 mM DTT, pH 9,6, 0-10 °C. Solubilization ratio: inclusion bodies (IB) isolated from 1 g of biomass were solubilized in 6 ml of solubilization buffer for 2 hours, at +4°C, and centrifuged thereafter at 9500 rpm for 30 min after this step.

Inclusion bodies (IB) washing were performed in a such manner:
a) Two times inclusion bodies were washed with washing buffer I (containing additives of 1 M NaCl, 0.1% Polysorbate-80);
b) The third washing with buffer II (containing additive of 6 M Urea);
c) The fourth washing with buffer III, 10 mM Tris-HCl buffer (without additives, pH 8,0). It was final IB pre-washing step;
d) Inclusion bodies after each of these washing steps were centrifuged in Beckman J-10 rotor, 9500 rpm, 30 min, 4°C.

Pre-washing ratio: 10 ml of buffer for IB isolated from 1 g wet biomass.

The refolding of the fusion with the additives of 0.5 M ammonium sulfate at a dilution factor of 32x was chosen as the main procedure for further purification scheme development. Albeit additives of sodium sulfate enable obtain higher recovery of the protein from Phenyl-Sepharose FF column, the use of sodium sulfate was postponed due to its propensity for crystallization at cold room conditions. Phenyl-Sepharose FF chromatography process was performed as above, in description of IFNa2b-IFNa5 purification. DEAE chromatography used two-buffer system: buffer A: 20mM Tris-HCl, pH 8.8, conductivity 243 µS/cm; and buffer B: 20mM Tris-HCl, 1M NaCl, pH 8,8, conductivity 84,4 mS/cm. Column was equilibrated with 20 mM Tris-HCl, 0.05 M NaCl, pH 8.8, conductivity 7 mS/cm, 25 °C, 5 CV. Elution was performed with 20 mM TrisHCl, gradient from 0.05 to 0.4 M NaCl, pH 8,8. Washing: 20 mM TrisHCl, 0,05 M NaCl, pH 8,8, conductivity 7 mS/cm, 25 °C, 2CV. Final chromatography step was the use of CHT Ceramic Hydroxyapatite type I sorbent. In this chromatography, buffer A was deionised water and buffer B: 500mM H3PO4, pH 6.4. Equilibration of the column was performed with 5mM H3PO4, pH 6.4. Fractions after DEAE chromatography diluted to a final concentration of 5mM Phosphate with 500mM Phosphate buffer (60:0.6) pH adjusted to pH 6.4. Desorption was performed with phosphate gradient from 5mM to 250mM pH 6.4.. Pooled fractions containing fusion were added to 1 part (10:1 volume) of concentrated acetate buffer (150 mM acetate, 150 mM NaCl, pH5.2), pH corrected to 5.2 and the solution was concentrated using Millipore Ultracel (10MWCO) centrifugal filters. Protein solution was filtered using Mustang E filter and aliquoted into vials for 1 ml (final concentration 1,005mg/ml) in sterile environment (Laminar airflow hood). Vials were frozen and kept at -80°C temperature. Samples were taken for RP-, SEC-HPLC, total protein and endotoxin determination.

Analysis consisted of in-process control methods (such as SDS-PAGE, Western blot, RP-HPLC) and final product characterization (such as RP-HPLC, SE-HPLC, peptide mapping and amino acid sequencing with TOF mass spectrometry and biological activity).

### EXAMPLES

### Example 1. IFNA5-IFNA5 homodimer cloning scheme

There are several backdraws in regular cloning schemes used for gene fusing when object is homodimeric gene. Different properties of gene A and gene B cannot be utilized in this particular case, and most of the strategies will result in the loss of the particular gene fragment. In this example, cloning scheme is presented (Fig. 1) and is can be used for the similar cases in homodimeric gene fusions.

IFNa5 gene preparation for fusion as N terminal part consisted of several stages. First, gene amplification with PCR, adding Ndel site on its 5' end and removing TAG stop codon from its 3' end (with primers IFNA5-FOR-NDE and IFNA5-REV-(-STOP)). Second, amplified and purified IFNA5/N gene was then digested with Acll restriction endonuclease, removing 22 bp from the 3' end of gene. Acll site was used for sticky end ligation with 3' end IFNa5/C gene.

IFNa5 gene preparation for fusion as C terminal part consisted of gene amplification with PCR, adding HindIII site on its 3' end and removing ATG start codon from its 5' end (with primers IFNA5-FOR-A5 and IFNA5-REV-HIND). Additional 30 bp sequence with complementary 3' end of IFNa5/N gene sequence was added upstream reading frame by using the same primer pair (see table 4). Amplified and purified IFNa5/C gene was then digested with Acll restriction endonuclease. It's first target is in added IFNa5/N sequence and the second target is on its 3' tail. Both Acll digested genes were ligated together with equimolar ratios to obtain a fused gene construct, afterwards extracting it from agarose gel.

**Table 3**

| Primer | Sequence (5'→3') | Restriction endonuclease site | Tm |
|---|---|---|---|
| IFNA5-FOR-NDE | ATTATCATATGTGTGATCTGCCGCAGA | NdeI (CATATG) | 63.9°C |
| IFNA5-REV-(-stop) | TTCCTTACGACGTAAACGTTCTTGC | AclI (AACGTT) | 60.8°C |
| IFNa5-A5-FOR | | AclI (AACGTT) | >75.0°C |
| IFNa5-REV-HIND | GACGTAAACGTTCTTGCTAAAAGCTTTTAA | Acl, HindIII | >75.0°C |

The underlined sequences in table 4 are Ndel recognition and cleavage site for forward primer (CA^ATG) and HindIII for reverse (A^AGCTT), Acll (AA^ACGTT) sites was used for gene connection. Bold sequence is 24 bases coding for IFNa5/N gene end added in front IFNa5/C gene.

After ligation, if concatamers are removed during clone selection stage, one obtains a fused gene product devoid of 22 bp (7 amino acids and no STOP codon). To restore a full sequence, a second stage consisting of digestion-ligation sequence was performed. A pET21b+ plasmid already carrying a IFNa5 gene was digested with unique restriction endonuclease within the IFNa5 gene - Bsu36I. Likewise, a IFNa5-AclI-IFNa5 ligate was also digested with Bsu36I and a 500bp internal gene fragment was purified from gel. The IFNa5/pET21b+ backbone is then ligated with 500bp Bsu36I IFNa5-IFNa5 fragment and transformed into transient E.coli JM109 strain. As it can be seen in cloning scheme (Figure 1), this pathway recreates full length of gene dimer with Start and Stop codons. Positive clones were additionally checked by performing full restriction pattern analysis. Confirmed clones were retransformed into BL21(DE3) E.coli strain and verified for their expression, as well as sequenced.

### Example 2. Cloning scheme of hetero fused construction IFNA2B-IFNA5

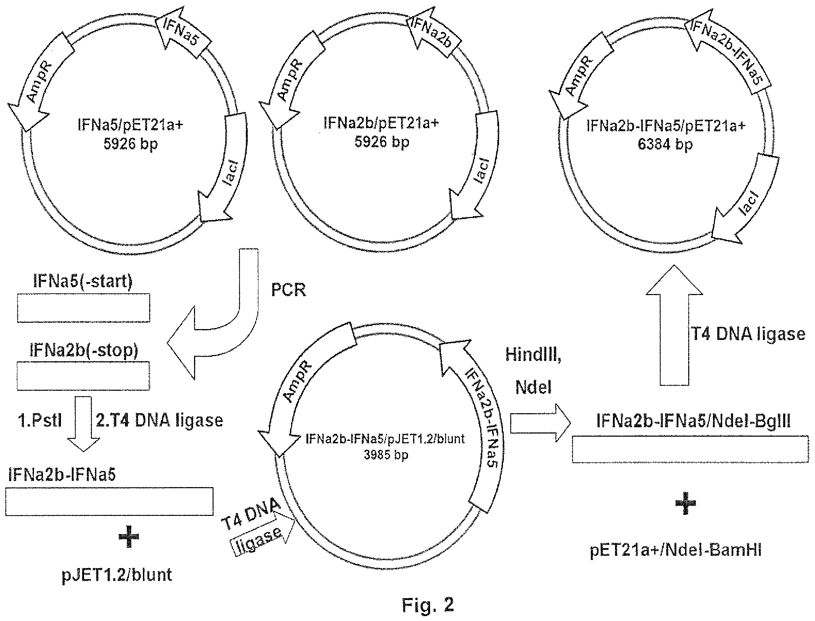

IFNa2b gene preparation for fusion consisted of gene amplification with PCR, adding Ndel site on its 5' end and removing TAA stop codon from its 3' end. This PCR is performed with primers IFNA2b-FOR-NDE and IFNa2B-REV-(-stop). Cloning scheme is presented in Figure 2. Amplified and purified IFNA2b gene was then digested with PstI restriction endonuclease, removing 10 bp from the 3' end of gene. PstI site was used for sticky end ligation with IFNa5 gene. Likewise, IFNa5 gene preparation for fusion consisted of gene amplification with PCR, adding HindIII site on its 3' end of gene and a flanking 22 bp length fragment of IFNa2b gene end with Pstl target (with primers IFNa5-FOR-A2b and IFNa5-REV-HIND). Amplified and purified IFNa5 gene was subsequently digested with PstI target in added IFNa2b sequence.

**Table 4**

| Primer | Sequence (5'→3') | Restriction endonuclease site | Tm |
|---|---|---|---|
| IFNA2b-FOR-NDE | TTAACATATGTGTGATCTGCCGCA | NdeI (CATATG) | 61.8°C |
| IFNa2B-REV-(-stop) | | PstI (CTGCAG) | 67.0°C |
| IFNa5-FOR-A2b | | PstI (CTGCAG) | 75.8°C |
| IFNa5-REV-HIND | AATTAAGCTTTTATTCCTTACGAC | HindIII (AAGCTT) | 51.3°C |

in the table 5, the underlined sequences are Ndel recognition and cleavage site for forward primer (CA^TATG) and HindIII for reverse (A^AGCTT), PstI (CTGCA^G) sites was used for gene connection. Bold sequence is 30 bases coding for IFNa2b gene end added in front of IFNa5 gene.

PstI digested products were ligated together with T4 DNA ligase in equimolar ratios. The resulting mix was purified, cutting the 1000 bp length fragment from the gel. This fragment is blunt-ended and thus was cloned into intermediate vector pJET1.2 and subsequently cloned into E.coli JM109 strain. This combination of plasmid and strain enabled to obtain positive clones in pJET plasmid which were then double-digested with Ndel-HindIII. Double-digested fused gene product was re-purified with PCR purification kit and was then prepared to clone into selected digested and dephosphorylated expression plasmid pET21b+. First round transformation of ligated products was performed into E.coli JM109 strain to guarantee clone stability. After first cloning, the size of the insert was checked by digesting plasmid DNA with Ndel-Hindlll restriction endonucleases. Positive clones were additionally checked by performing full restriction pattern analysis. Confirmed clones were retransformed into BL21(DE3) E.coli strain and verified for their expression level. Confirmed clones were sequenced.

### Example 3. Cloning scheme of hetero fused construction IFNA5-IFNA2B

IFNa5 gene preparation for fusion consisted of gene amplification with PCR, adding Ndel site on its 5' end and removing stop codon from its 3' end with primers IFNA5-FOR-NDE and IFNAS-REV-(-stop). Likewise, IFNa2b gene preparation for fusion consisted of gene amplification with PCR, adding HindIII site on its 3' end of gene and a flanking 14 bp length fragment of IFNa5 gene (primers IFNa2b-FOR-A5 and IFNa2b-REV-HIND). In this case, an overlapping PCR was performed via indicated 14bp region that resulted in fused gene product. Primers are presented in the table below.

**Table 5**

| Primer | Sequence (5'→3') | Restriction endonuclease site | Tm |
|---|---|---|---|
| IFNA5-FOR-NDE | ATTATCATATGTGTGATCTGCCGCAGA | NdeI (CATATG) | 63,9°C |
| IFNA5-REV-(-stop) | TTCCTTACGACGTAAACGTTCTTGC | AclI (AACGTT) | 60.8°C |
| IFNA2B-FOR-A5 | TACGTCGTAAGGAATGTGATCGCCGCA | none | 68.9°C |
| IFNA2B-REV-HIND | AAAAAGCTTCTATTCCTTAGAAC | HindIII (AAGCTT) | 50.2°C |

Overlapping PCR was performed in two stages: one stage of low yield PCR, when reaction is performed without primers (overlapping region acts as donor of free 3'-OH groups) and only PCR products of IFNa5 and IFNa2b are included. After gel-extraction of fused gene product, it is reamplified using primers IFNA5-FOR-NDE and IFNA2B-REV-HIND. After this second stage of PCR, the product is digested with restriction endonucleases Ndel and Hindlll and is cloned into digested and dephosphorylated expression plasmid pET21b+. First round transformation of ligated products was performed into E.coli JM109 strain to guarantee clone stability. After first cloning, the size of the insert was checked by digesting plasmid DNA with Ndel-HindIII restriction endonucleases. Positive clones were additionally checked by performing full restriction pattern analysis. Confirmed clones were retransformed into BL21(DE3) E.coli strain and verified for their expression level. Confirmed clones were sequenced.

### Example 4. Cloning scheme of hetero fused construction IFNa5-OSM

IFNa5 gene preparation for fusion consisted of gene amplification with PCR, adding NdeI site on its 5' end and removing TAG stop codon from its 3' end (with primers IFNA5-FOR-NDE and IFNAS-REV-(-stop)). Amplified and purified IFNa5 gene was then digested with AclI restriction endonuclease, removing 10 bp from the 3' end of gene. AclI site was used for sticky end ligation with OSM(196) gene.

OSM(196) gene preparation for fusion consisted of gene amplification with PCR, adding HindIII site on its 3' end of gene and a flanking 22 bp length fragment of IFNa5 gene end with Acll target. Amplified and purified OSM(196) gene was then digested with AclI target in added IFNa5 sequence.

**Table 6**

| Primer | Sequence (5'→3') | Restriction endonuclease site | Tm |
|---|---|---|---|
| IFNA5-FOR-NDE | ATTATCATATGTGTGATCTGCCGCAGA | NdeI (CATATG) | 63.9°C |
| OSM-RSRR-REV-HIND | TTTAAGCTTCTATCTCCGGCTCCGGTT | HindIII (AAGCTT) | 65.1°C |
| IFNA5-REV-(-stop) | TTCCTTACGACGTAAACGTTCTTGC | AclI (AACGTT) | 60.8°C |
| OSM-AAI-ACL | | AclI (AAGCTT) | 71.5°C |

The underlined sequences are Ndel recognition and cleavage site for forward primer (CA^TATG) and HindIII for reverse (A^AAGCTT), Acll (AA^CGTT) sites was used for gene connection. Bold sequence is 24 bases coding for IFNa5 gene end added in front of OSM(196) gene.

AclI digested products were ligated together with T4 DNA ligase in equimolar ratios. The resulting mix was purified, cutting a 1100 bp length fragment from agarose gel. The main difference from Example 2 is that due to different genes to be fused sizes, concatameric constructions (mirrored IFNa5-IFNa5 and OSM-OSM) can be separated from correct form. Thus, intermediate pJET1.2 cloning stage is not necessary and can be omited. Extracted from agarose gel DNA fragment was digested with Ndel and HindIII and cloned into a accordingly digested pET21b+ plasmid and transformed into JM109 E.coli strain. Ampicilin-resistant clones were subjected for insert-size analysis. Selected clones were retransformed into E.coli strains BL21(DE3) and a full restriction pattern analysis was performed on their plasmid DNA. Confirmed clones were sequenced.

### Example 5 Cloning scheme of hetero fused construction OSM-IFNa5

OSM gene preparation for fusion consisted of gene amplification with PCR, adding Ndel site on its 5' end and removing TAG stop codon from its 3' end. Amplification of PCR product was performed with forward primer OSM-FOR-NDE and reverse primer OSM-REV-(-STOP). Amplified and purified OSM gene was then digested with BsaWI restriction endonuclease, removing 10 bp from the 3' end of gene. BsaWI site was used for sticky end ligation with IFNa5 gene.

IFNa5 gene preparation for fusion consisted of gene amplification with PCR, adding HindIII site on its 3' end of gene and a flanking 25 bp length fragment of OSM gene end with BsaWI target. Amplification of PCR product was performed with forward primer IFNA5-FOR-BSA and reverse primer IFNA5-REV-HIND. Amplified and purified IFNA5 gene was then digested with BsaWI target in added OSM sequence.

**Table 7**

| Primer | Sequence (5'→3') | Restriction endonuclease site | Tm |
|---|---|---|---|
| OSM-FOR-NDE | AAACATATGGCGGCTATAGGCAGC | NdeI (CATATG) | 66.2°C |
| OSM-REV-(-STOP) | TCTCCGGCTCCGGTTCGG | BsaWI (ACCGGA) | 65.7°C |
| IFNAS-FOR-BSA | | BsaWI (ACCGGA) | >7S°C |
| IFNA5-REV-HIND | AATTAAGCTTTTATTCCTTACGAC | HindIII (AAGCTT) | 51,3°C |

BsaWI digested products were ligated together with T4 DNA ligase in equimolar ratios. The resulting mix was purified, cutting a 1100 bp length fragment from agarose gel. Extracted from agarose gel DNA fragment was digested with NdeI and HindIII and cloned into a accordingly digested pET21b+ plasmid and transformed into JM109 E.coli strain. Ampicitin-resistant clones were subjected for insert-size analysis. Selected clones were retransformed into E.coli strains BL21(DE3) and a full restriction pattern analysis was performed on their plasmid DNA .Confirmed clones were sequenced.

### Example 6. BIOSYNTHESIS OF IFNA2B-IFNA5/PET21B+/E.COLI BL21(DE3) IN FERMENTOR

In all studied cases batch mode biosynthesis of E. coli BL21(DE3)/ pET21b+/IFNa2b-IFNa5 was performed in 1.4 L working volume fermentor "Biostat M" at pH 6.8-7.0, pO2 - 30% with automatically controlled on-line variables (temperature, stirring, pH, pO2) and off-line variable - optical density (abbr. OD, measured at 600nm wavelength with Eppendorf spectrophotometer).

Two media for cell cultivation were used - M9 and modified M9m. M9 medium (for fermentation) composition (g/L): yeast extract - 20.0, ammonium chloride - 5.0, magnesium sulfate heptahydrate - 0.5, di-sodium hydrogen phosphate - 4.7, potassium dihydrogen phosphate - 4.5, glucose monohydrate - 22. M9m medium (for inoculum) composition (g/L): yeast extract - 10, ammonium chloride - 1, magnesium sulfate heptahydrate - 0.5, di-sodium hydrogen phosphate - 6,6, potassium dihydrogen phosphate - 2,7, glucose monohydrate - 5. M9m medium (for fermentation) composition (g/L): yeast extract - 20.0, ammonium chloride - 5.0, magnesium sulfate heptahydrate - 0.5, di-sodium hydrogen phosphate - 7, potassium dihydrogen phosphate - 6,8, glucose monohydrate - 15.

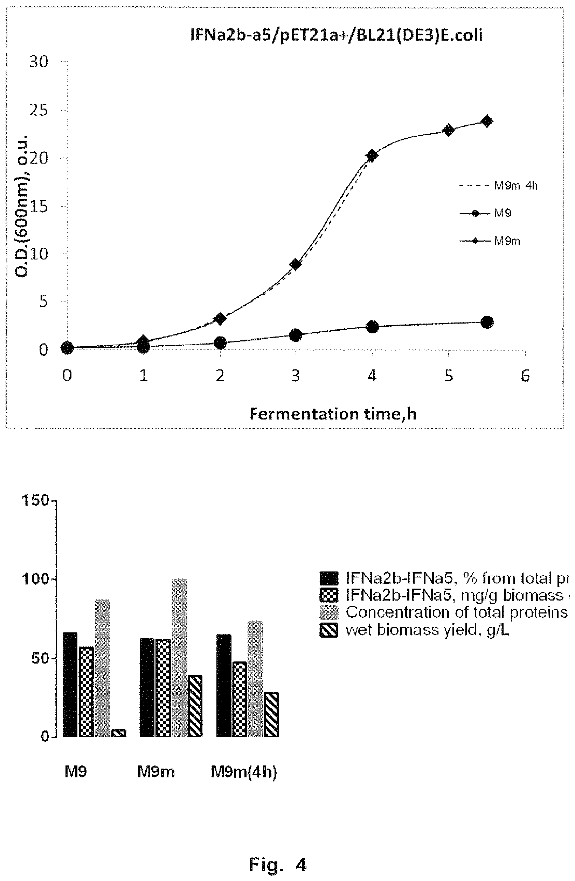

The comparison of fermentation processes in these two media is presented in the table 8 below and Figure 4. It can be observed that modified M9 medium is significantly superior in all controlled parameters when compared to M9 medium. Initial duration of biosynthesis experiment was 5.5 hours, with the induction at the 3rd hour of cultivation.

**Table 8**

| Controlled parameters | M9 medium | M9m medium |
|---|---|---|
| Working volume, L | 1.4 | 1.4 |
| Final IPTG concentration, mM | 0.5 | 0.5 |
| Fermentation duration, h | 5.5 | 5.5 |
| Induction time, h | 3.0 | 3.0 |
| Induction at OD | 1.5 | 8.9 |
| Final OD | 2.9 | 23.9 |
| Yield of wet biomass, 9/L | 4.6 | 39.3 |
| Total proteins, mg/g wet biomass | 86.6 | 99.6 |
| IFNa2b-IFNa5, % of total proteins | 66.0 | 62.5 |
| IFNa2b-IFNa5, mg/g biomass (calculated acc. total proteins) | 57.1 | 62.2 |
| IFNa2b-IFNa5, mg/L cell suspension (acc. total proteins) | 262.8 | 2445.9 |

However, the duration of 5.5 hours of biosynthesis resulted in decrease of plasmid stability in last hour period (plasmid stability was only 60% at the endpoint). Accumulation of the cells with no plasmid is unwanted because it increases the protein burden during purification. To avoid this problem, biosynthesis process was changed in time schedules - induction point shifted to the second hour of cultivation, and leaving only 2 hours of cultivation after induction. Optimized biosynthesis in M9m medium produced following results for in process control analytics:

**Table 9**

| Controlled parameters | Value |
|---|---|
| Yield of wet biomass, g/L | 28.5±0.9 |
| Total proteins, mg/g wet biomass | 73.4±7.6 |
| IFNa2b-IFNa5, % from total proteins (SDS-PAGE) | 65.1±10.2 |
| IFNa2b-IFNa5, mg/g biomass (calculated acc. total proteins) | 47.7±8.5 |
| IFNa2b-IFNa5, mg/L cell suspension (acc. total proteins) | 1364.1±72.8 |

### Example 7. ISOLATION AND PURIFICATION OF IFN ALPHA-2B-IFN ALPHA-5 FUSION

Chromatography purification steps were as follows: hydrophobic interaction chromatography over Phenyl-Sepharose, further anion- and cation-exchange chromatography; however each of these steps was also properly adjusted with respect to the behaviour of the fusion protein.

For process development purposes 10 g of biomass was used as a starting material. 10g of biomass was suspended in a buffer solution composed of 89.6 ml of 0.1 M Tris-HCl buffer pH 8.0 2 mM FDTA and 25 mM DTT, 400 µl of 250 mM PMSF and 10 ml of 0,1% TritonX-100. pH value is adjusted to 7.5 adding 6 M HCl. Cells are disrupted with Sonics Vibra cell sonicator for 30 min with impulses of 1 second - on, 2 seconds - off, with 40% power and thereafter soluble and insoluble proteins were separated by centrifugation at 12000rpm for 30 min at +4 °C. Inclusion bodies are washed twice with cold (+4°C) buffer-I containing additives of 1 M NaCl and 0.1% polysorbate-80. For the third washing is used buffer-II containing 6 M urea, and washing procedure is finished with a buffer-III. After each pre-washing cycle IB were suspended in 100 ml of respective buffer at a ratio 10 ml bufer for IB isolated from 1g of biomass and centrifuged. Washing buffers composition: buffer-I: 10 mM Tris-HCl, 1 M NaCl, 0,1 % Polysorbate-80, pH 7.4, buffer-II: 10 mM Tris-HCl, 6 M urea, pH 8.0, buffer-III: 10 mM Tris-HCl, pH 8.0. Pre-washed inclusion bodies were suspended in 60 mL of 25 mM Gly/NaOH buffer pH 9.6 containing 6 M guanidine hydrochloride (GdmCl); and 20 mM DTT. Solubilization was performed by continuous stirring for 2 hours at +4⁰C. After that the solubilisate of IB was centrifuged at 12 000 rpm for 30 min at +4 °C

In all studied cases the refolding was performed by total 16x dilution of IB solution. For this purpose 56 ml of IB solution was poured into refolding buffer by gentle mixing final solution for defined duration (40-120 hours) at +4 °C. Refolding buffer was composed of 25 mM glycine/NaOH buffer of pH 9.6 value containing 1.2 M NaCl, 0,5 M L-Arg, and selected pair of reduced/oxidised glutathione GSH/GSSG = 5.0mM:0,25mM.

The first chromatography purification step was chromatography of refolded fusion over Phenyl-Sepharose FF column. This chromatography step is mainly dedicated for separation of correctly folded protein from residual (0.375 M) GdmCl which presence restricted possibilities to use other conventional chromatography purification procedure of recombinant proteins. So, the solution of the fusion protein after refolding step was loaded onto Phenyl-Sepharose FF column in 20 mM TrisHCl buffer pH 8.2 containing 1.5 M NaCl and eluted from the column with 10 mM TrisHCl buffer pH 9.2 without added salts. Eluted protein was further chromatographed over anion-exchangers. Here, we tested Q-Sepharose FF media and DEAE-Sepharose FF anion-exchanger. For the third chromatography purification SP-Sepharose FF column was chosen with pH 5.5. The fusion recovered from this column was splited into two parts, the one of which was in acetate buffer system and the other was buffer exchanged to PBS and both protein parts were concentrated up to 1 mg/ml, sterile-filtered and aliquoted for stability evaluation during freezing at -80°C and freezing-thawing cycle. Both formulations proved to be stable.

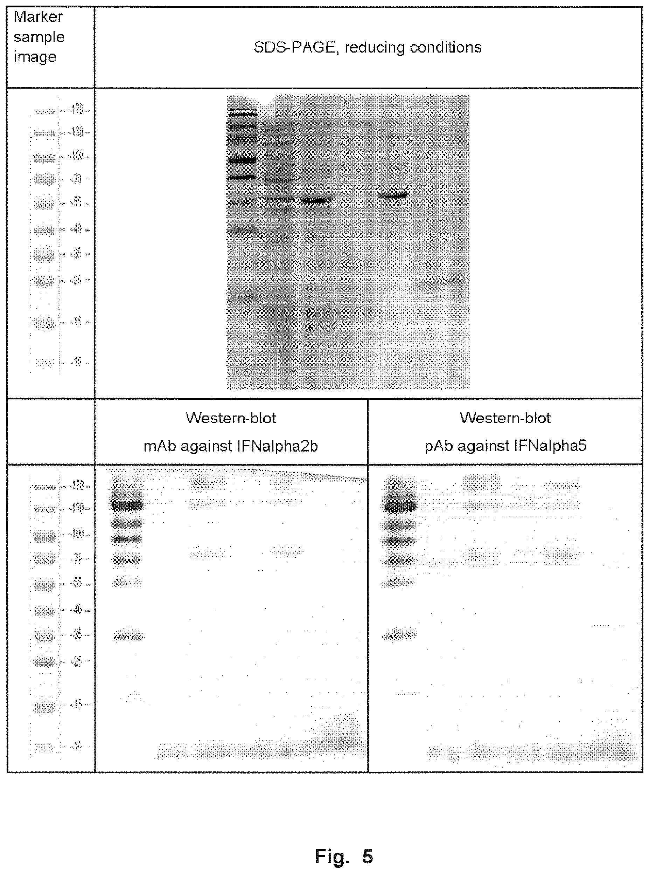

The data from Mass spectrometer evidenced that the fusion composed from IFN alpha-2b and IFN alpha-5 partners contained correct polypeptide chain with N-terminus containing the sequence of IFN alpha-2b. Determined molecular mass of the fusion is in good accordance with calculated molecular mass. Both partners of the fusion recognized respective specific antibodies (Figure 5) as an indication that both partners are correctly folded. In sum, the data presented in this example evidenced that genetical fusion of IFN alpha-2b with IFN alpha-5 gives the new entity which can be produced with acceptable quality attributes for proof-of-concept studies.

### Example 8. DETERMINATION OF CHIMERA'S PURITY BY SE-HPLC

The level of chimera's monomer in purified sample was semi-quantitatively determined using SE-HPLC method. No sample preparations was carried out.

The amount of sample injection were evaluated according to absorption mean of fraction of chromatography. The main peak of chimera was identified using MS analysis.

SE-HPLC experimental details:
Mobile phase: A - 50mM Sodium phosphate, pH7.0, 0.4M Sodium chloride
Column: Tosoh TSKgel30WXL 7.8x300 mm, particle size 5 µm
Chromatography system: HPLC system Alliance, Waters
Wavelength of detection: 280 nm
Flow rate: 1ml/min

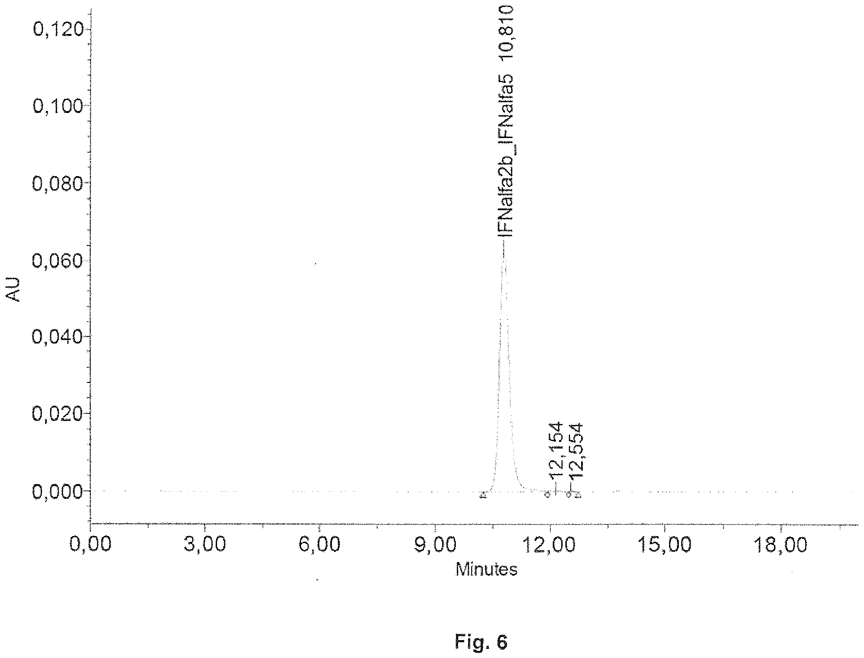

Results of SE-HPLC of purified IFNa2b-IFNa5 fusion protein are presented in Fig. 6. It can observed from this chromatographic view that a monomeric content of fusion protein is more than 98%.

### Example 9. PEPTIDE MAPPING

25 µl of Test solution (1mg/ml) was added into 200 µl test tube with 2.8 µl 1,0 mg/ml trypsin solution. 1.6 µl of 1.0 M Potassium phosphate buffer, pH 8.0; then 3.6 µl of water were added and the content of the test tube stirred thoroughly. Centrifugation at 6000 rpm for 5 seconds was followed by incubation of the solutions in Thermomixer Comfort at 37 °C temperature for 18 hours. After incubation 100 µl of 6.0 M Gu-HCl solution was added into test tube, following by addition of 7 µl of 1.0 M DTT solution. The test tube was placed into the boiling water for 1 minute, and then the prepared samples were allowed to cool to the room temperature.

**Table 10**

| Parameter | Value (notes) | |
|---|---|---|
| UV detector | Wavelength | 214 nm |
| Temperature | Column | 30 °C |
| | Sample storage | 6 °C |
| Solvent manager | Channel of mobile phase A | Mobile phase A (Deionized water with 0.1 % TFA) |
| | Channel of mobile phase B | Mobile phase B (Acetonitrile with 0.1% TFA) |
| | Mobile phase flow rate | 1 ml/min |

**Table 11**

| HPLC system program | | | | |
|---|---|---|---|---|
| No. | Time, min. | A, % | B, % | Notes |
| 1 | 0,01 | 100,0 | 0,0 | Start |
| 2 | 8,00 | 100,0 | 0,0 | Isocratic |
| 3 | 68,00 | 40,0 | 60,0 | Linear gradient |
| 4 | 72,00 | 40,0 | 60,0 | Isocratic |
| 5 | 75,00 | 100,0 | 0,0 | Linear gradient |
| 6 | 90,00 | 100,0 | 0,0 | Equilibration |

| | | | | |
|---|---|---|---|---|
| HPLC column: Waters Symmetry 3000 C18 4.6x250mm | | | | |

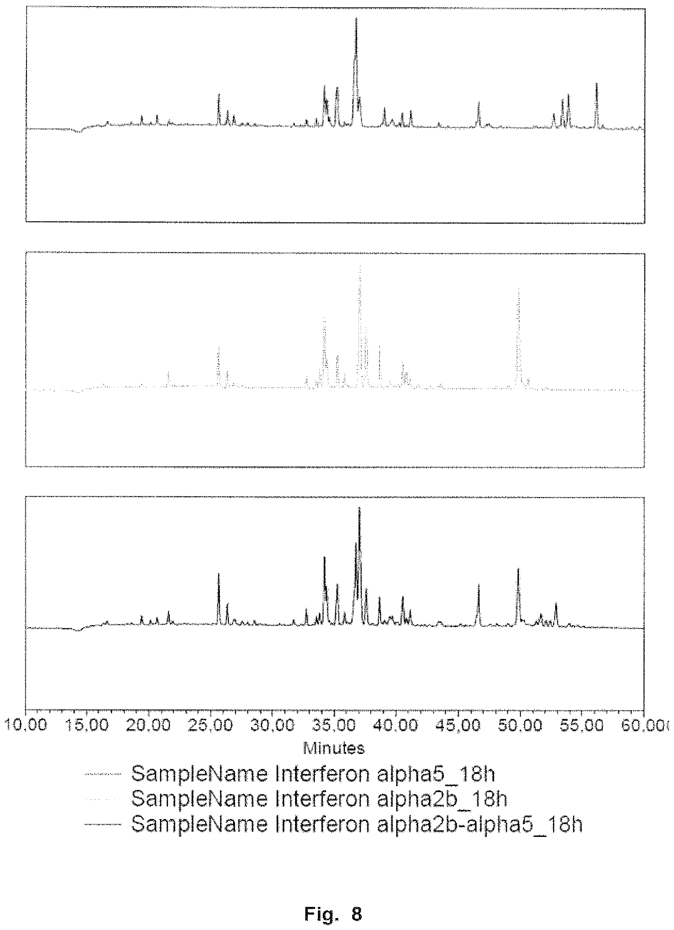

Results of peptide mapping are presented in Figure 8. From HPLC chromatograms it is obvious that a new entity of fusion protein exhibits all the peaks present in peptide mapping of its individual partners.

### Example 10. BIOLOGICAL ACTIVITY OF SELECTED IFNA-5 FUSED PROTEINS

Human negroid cervix carcinoma cells Hep2c and murine encephalomyocarditis virus (EMCV) were obtained from ATCC collection. Hep2c cells were cultured in RPMI 1640 supplemented with 10% heat-inactivated neonatal calf serum and 1.5 g/L sodium bicarbonate. Cells were maintained at a density of (5-6) × 10⁵ cells. EMCV was propagated in mouse fibroblast L-929 cells.

The biological antiviral activity of interferon alpha 5 and its fused proteins were established by performing EMCV cytopathic effect (CPE) assay. The virus was titrated by plaque formation on Hep2c cells. The principle of the method is described (Armstrong, 1971). All viral dilutions were made in RPMI 1640 medium supplemented with 2% heat-inactivated fetal bovine serum. Hep2c cells were seeded in a 96-well plate 1 day prior to infection at a concentration of 5-6 × 10⁵ cells/mL; simultaneously the cells were treated in triplicate with 50 µL of culture medium containing various concentrations of chimeric IFNα preparations. After incubation at 37°C in 5% CO2 for 20-24 h Hep2c were infected with 20 plaque-forming units of EMCV. Controls wells were treated with EMCV and Hep2c without IFN, Hep2c alone and medium alone. After 24 hr of incubation, the virus controls should show 90 to 100% CPE as observed microscopically, and incubation may be terminated. Occasionally a longer incubation may be needed. The medium from each well was aspirated, and the cells were stained with 0.05% (w/v in 20% ethanol) crystal violet solution. After 30 min, the crystal violet solution was decanted and the wells were gently rinsed with water, dried and eluting solution (50% ethanol with 0.05% acetic acid) was added. The absorbance of the crystal violet-stained wells were determined in a microplate reader (µQuant, BioTek) at 570 nm.

The biological activity of the various preparations of IFNa5 was calculated on the basis of International standard of Interferon alpha-2b (human rDNA derived), NIBSC Product No. 95/566, 70000 IU/ampoule and expressed in international units (IUs). The controls (no IFN, no EMCV) were assigned protection values of 0% and 100%, respectively. Interferon effect was scored in cups having 50% less degeneration than virus controls.

Results of the cytopathic effect reduction assay generally fited a sigmoidal dose-response curve, when the interferon concentration (the log of the reciprocal of the interferon dilution) was plotted versus stain absorbance. Interferon concentration (log reciprocal of dilution) was plotted versus the stain absorbance for the interferon reference preparation calibrated in International Units and for the interferon test solutions. Using the linear portion of the curve, the concentration of interferon in the sample was calculated by comparing the responses for test and reference solutions. Statistical program PLA version 2.0 was used for a parallel line assay for biological potency calculations of all experimental samples. All calculations were performed on the basis of test results obtained per 2 days × 2 plates per day (standard and test solutions titration performed in triplicate). Results are presented in the table below. It can be observed, that all three fused variants exhibited a biological activity that is characteristic to IFNa5.

**Table 12**

| Interferon-α preparation | Concentration, mg /ml | Antiviral bioactivity, × 10⁸ IU/mg |
|---|---|---|
| IFNa2b | 8.11 | 1.77 |
| IFNa5 | 8.79 | 0.72 |
| Mixture of IFNa5 and IFNa2b | 8.45 | 1.24 |
| IFNa2b-IFNa5 | 1.30 | 0.07 |
| IFNa5-IFNa5 | 1.53 | 0.29 |
| IFNa5-OSM | 0.98 | 0.21 |

In addition to maintained biological activity of fused IFNa5 proteins, fused proteins were tested for activity as antiviral agents in infected rats. In these assays, one unit is defined as the quantity of interferon required per milliliter to reduce the cytopathic effect of viral infection by 50%. For example, IFNa5 in rat serum, IU/ml is observed for 6 hours, with range from 208 IU/ml after 0.5 hour after injection to 61.22 IU/ml after six hours. IFNa2b-IFNa5 fused protein ranges from 191 IU/ml (0.5h) to 54 IU/ml (6 h), and IFNa5-IFNa5 homodimer is observed for 2 hours longer, up to 8 hours, with constant activity of 40-46 IU/ml during all tested 0.5-8 hour period.

From this study, we can see that some of IFNa5 fused proteins have even better properties when comparing to IFNa5. For example, IFNa2b-IFNa5 has the same antiviral activity in rats even though it displayed rather low *in vitro* biological activity. IFNa5-IFNa5 exhibits prolonged antiviral action in rats.

### Industrial applicability

In this patent, we present a versatile platform for viral infection and cancerous disease treatment: IFNa5 fused proteins. Depending on the partner cytokine, these proteins can be recruited for a wide range disease treatment, including viral infections, liver diseases and cancers. A presented invention covers entity of methods that are suitable to clone, cultivate, purify and formulate a protein that can be then used for wide range of animal based studies and preclinical trials.

## Claims

1. Fused protein, comprising an interferon alpha 5 (IFNa5), of a general formula (I) or (II)
IFNa55 - X (I)
X - IFNa5 (II)
wherein fusion partner X is another alpha interferon or cytokine.

2. Fused protein according to claim 1, wherein IFNa5 is human recombinant interferon alpha 5, and X is selected from the group, consisting of IFNa5, INFa2b and oncostatin.

3. Fused protein according to claim 1 or 2, which is IFNa5-IFNa5 homodimer with amino acid sequence of SEQ ID No: 1 or at leat 95% identical sequence.

4. Fused protein according to claim 1 or 2, which is IFNa2b-IFNa5 heterodimer with amino acid sequence of SEQ ID No: 2 or at least 95% identical sequence.

5. Fused protein according to claim 1 or 2, which is IFNa5-IFNa2b heterodimer with amino acid sequence of SEQ ID No: 3 or at least 95% identical sequence.

6. Fused protein according to claim 1 or 2, which is IFNa5-OSM heterodimer with amino acid sequence is SEQ ID No: 4 or at least 95% identical sequence.

7. Fused protein according to claim 1 or 2, which is OSM-IFNa5 heterodimer, which amino acid sequence is SEQ ID No: 5 or at least 95% identical sequence.

8. Fused protein according to any claim of 1-7, exhibiting a biological actyvity, characteristic to IFNa5 along with prolonged clearance rate.

9. DNA fragment, encoding fused protein as defined in claims 1-8, wherein coding sequence is selected from the group, consisting of SEQ ID NO: 6, SEQ ID NO: 7, SEQ ID NO: 8, SEQ ID NO: 9, SEQ ID NO: 10 or is the sequence, coding the same protein.

10. Vector, comprising DNA fragment according to claim 9, encoding fused protein as defined in claims 1-8, wherein said vector is selected from the group, consisting of pET21b+, pET28a+, pUC57/T and pT7, preferably is pET21b+.

11. A process for producing a fused protein as defined in claims 1-8 by expression in an appropriate host cell, comprising:
a) providing of host cell, producing said IFNa5 fused protein;
b) cultivating host cells under conditions effective to express said IFNa5 fused protein in a suitable fermentation medium;
c) isolating and purifying the expressed IFNa5 fused protein, ***characterised* in that**:
- said isolation is isolation from the obtained in step b) mixture, comprising the target fused protein in a form of inclusion bodies, by subjecting them to solubilization and following oxidizing renaturation; and
- said purification is 3-step chromatographic treatment comprising:
subjecting said mixture comprising renatured IFNa5 fused protein to a hydrophobic interaction chromatography;
following by subjecting the solution obtained in the previous step to an anion-exchange chromatography;
and further subjecting the solution obtained in the previous step to a cation-exchange chromatography.

12. The process according to claim 11, wherein host cell is selected from the group, consisting of E.coli, S.cerevisiae, K.lactis, preferably E.coli, more preferably E.coli protease deficient strain, such as E.coli BL21 and Rosetta gami-2, most preferably an E.coli BL21, and wherein suitable fermentation medium is any complex medium containing yeast extract as nitrogen source and glucose as carbon source, preferably M9m medium.

13. The process according to claims 11-12, wherein said conditions effective to express said IFN fused protein comprises induction, preferably with isopropyl-β-D-thiogalactopyranoside, preferably IPTG at concentration of 0.1 mM to 1 mM, most preferably 0.5 mM, and induction is optionally shifted to early growth stage.

14. The process according to claims 11-13, wherein said solubilization is performed in chaotropic and denaturing solution, comprising of 6-8 M urea or 4-6 M guanidine hydrochloride, preferably 6 M guanidine hydrochloride, optionally with 20 mM DTT.

15. The process according to claims 11-14, wherein said oxidising renaturation is performed in glycine/NaOH buffer at pH in the range of 7,0 - 9,6 and pair of reduced/oxidised glutathione GSH/GSSG in the ratio 5-20:1 with buffering additives, selected from the group, comprising sodium chloride, sodium sulphate and ammonium sulphate.

16. The process according to claims 11-15, wherein in said 3-step chromatographic purification the hydrophobic interaction chromatography sorbent is selected from the group, consisting of Butyl-Sepharose, Octyl-Sepharose, Phenyl-Sepharose, preferably Phenyl-Sepharose in pH range of 8,2-9,2;
the anion-exchange chromatography sorbent is selected from the group, consisting of ANX Sepharose, Q-Sepharose, DEAE-Sepharose, QAE-Sepharose, preferably Q-Sepharose or DIEAE-Sepharose in pH range of 8,6-9,0; and
the cation-exchange chromatography sorbent is selected from the group, consisting of S-Sepharose, SP-Sepharose and CM-Sepharose, preferably SP-Sepharose or CM-Sepharose in pH range of 5,2-5,6.

17. Fused protein as defined in claims 1-7, obtained by the process according to claims 11-16.

18. Preparation, comprising the fused protein according to claim 17 in the form of stable solution.

19. Fused protein according to claim 17 or preparation according to claim 18 for use in therapy.

20. Pharmaceutical composition, comprising therapeutically effective amount of fused protein according to claims 1-8 or 17 or preparation according to claim 18, and pharmaceutically acceptable carrier, excipient and/or auxiliary substances.

## Patentansprüche

1. Fusionsprotein, umfassend ein Interferon alpha 5 (IFNa5) der allgemeinen Formel (I) oder (II)
IFNa5-X (I)
X-IFNa5 (II),
wobei der Fusionspartner X ein weiteres alpha-Interferon oder Cytokin ist.

2. Fusionsprotein nach Anspruch 1, wobei IFNa5 humanes rekombinantes Interferon alpha 5 ist und X ausgewählt ist aus der Gruppe bestehend aus IFNa5, INFa2b und Oncostatin.

3. Fusionsprotein nach Anspruch 1 oder 2, das IFNa5-IFNa5-Homodimer mit der Aminosäuresequenz von SEQ ID NO: 1 oder einer zumindest 95% identischen Sequenz ist.

4. Fusionsprotein nach Anspruch 1 oder 2, das IFNa2b-IFNa5-Heterodimer mit der Aminosäuresequenz von SEQ ID NO: 2 oder einer zumindest 95% identischen Sequenz ist.

5. Fusionsprotein nach Anspruch 1 oder 2, das IFNa5-IFNa2b-Heterodimer mit der Aminosäuresequenz von SEQ ID NO: 3 oder einer zumindest 95% identischen Sequenz ist.

6. Fusionsprotein nach Anspruch 1 oder 2, das IFNa5-OSM-Heterodimer mit der Aminosäuresequenz von SEQ ID NO: 4 oder einer zumindest 95% identischen Sequenz ist.

7. Fusionsprotein nach Anspruch 1 oder 2, das OSM-IFNa5-Heterodimer ist, wobei die Aminosäuresequenz SEQ ID NO: 5 oder eine zumindest 95% identische Sequenz ist.

8. Fusionsprotein nach einem der Ansprüche 1 bis 7, das eine für IFNa5 charakteristische biologische Wirksamkeit zusammen mit einer verlängerten Clearance-Rate aufweist.

9. DNA-Fragment, das ein Fusionsprotein nach den Ansprüchen 1 bis 8 codiert, wobei die codierende Sequenz ausgewählt ist aus der Gruppe bestehend aus SEQ ID NO: 6, SEQ ID NO: 7, SEQ ID NO: 8, SEQ ID NO: 9, SEQ ID NO: 10 oder sie die Sequenz ist, die das gleiche Protein codiert.

10. Vektor, umfassend ein DNA-Fragment nach Anspruch 9, das für ein Fusionsprotein nach den Ansprüchen 1 bis 8 codiert, wobei der Vektor ausgewählt ist aus der Gruppe, bestehend aus pET21b+, pET28a+, pUC57/T und pT7, vorzugsweise pET21b+ ist.

11. Verfahren zur Herstellung eines Fusionsproteins nach den Ansprüchen 1 bis 8, durch Expression in einer geeigneten Wirtszelle, umfassend:
a) Bereitstellen einer Wirtszelle, Herstellen des IFNa5-Fusionsproteins;
b) Züchten von Wirtszellen unter Bedingungen, die wirksam sind, um das IFNa5-Fusionsprotein in einem geeigneten Fermentationsmedium zu exprimieren;
c) Isolieren und Reinigen des exprimierten IFNa5-Fusionsproteins, ***dadurch gekennzeichnet,* dass**:
- die Isolierung die Isolierung aus dem in Schritt b) erhaltenen Gemisch ist, das das Ziel-Fusionsprotein in Form von Einschlusskörpern enthält, indem man es einer Solubilisierung und nachfolgender oxidierender Renaturierung unterwirft; und
- die Reinigung eine 3-stufige chromatographische Behandlung ist, umfassend:
Unterwerfen des Gemischs, das renaturiertes IFNa5-Fusionsprotein enthält, einer hydrophoben Interaktionschromatographie;
anschließend Unterwerfen der im vorhergehenden Schritt erhaltenen Lösung einer Anionenaustauschchromatographie;
und ferner Unterwerfen der im vorhergehenden Schritt erhaltenen Lösung einer Kationenaustauschchromatographie.

12. Verfahren nach Anspruch 11, wobei die Wirtszelle ausgewählt ist aus der Gruppe, bestehend aus E. coli, S. cerevisiae, K. lactis, vorzugsweise E. coli, stärker bevorzugt einem Protease-defizienten E. coli-Stamm, wie E. coli BL21 und Rosetta gami-2, am stärksten bevorzugt einem E. coli BL21, und wobei das geeignete Fermentationsmedium ein beliebiges komplexes Medium ist, das Hefeextrakt als Stickstoffquelle und Glucose als Kohlenstoffquelle enthält, vorzugsweise M9m-Medium.

13. Verfahren nach den Ansprüchen 11 bis 12, wobei die Bedingungen, die wirksam sind, um das IFN-Fusionsprotein zu exprimieren, die Induktion, vorzugsweise mit Isopropyl-β-D-thiogalactopyranosid, vorzugsweise IPTG bei einer Konzentration von 0,1 mM bis 1 mM, am stärksten bevorzugt 0,5 mM, umfassen, und die Induktion wahlweise zum frühen Wachstumsstadium verschoben wird.

14. Verfahren nach den Ansprüchen 11 bis 13, wobei die Solubilisierung in einer chaotropen und denaturierenden Lösung durchgeführt wird, die 6 bis 8 M Harnstoff oder 4 bis 6 M Guanidinhydrochlorid, vorzugsweise 6 M Guanidinhydrochlorid, gegebenenfalls mit 20 mM DTT, umfasst.

15. Verfahren nach den Ansprüchen 11 bis 14, wobei die oxidierende Renaturierung in Glycin/NaOH-Puffer bei einem pH-Wert im Bereich von 7,0 bis 9,6 und einem Paar von reduziertem/oxidiertem Glutathion GSH/GSSG im Verhältnis 5-20:1 mit Pufferadditiven, ausgewählt aus der Gruppe, bestehend aus Natriumchlorid, Natriumsulfat und Ammoniumsulfat durchgeführt wird.

16. Verfahren nach den Ansprüchen 11 bis 15, wobei bei der 3-stufigen chromatographischen Reinigung das hydrophobe Interaktionschromatographie-Sorptionsmittel ausgewählt ist aus der Gruppe, bestehend aus Butyl-Sepharose, Octyl-Sepharose, Phenyl-Sepharose, vorzugsweise Phenyl-Sepharose im pH-Bereich von 8,2-9,2;
das Anionenaustauschchromatographie-Sorptionsmittel ausgewählt ist aus der Gruppe, bestehend aus ANX-Sepharose, Q-Sepharose, DEAE-Sepharose, QAE-Sepharose, vorzugsweise Q-Sepharose oder DEAE-Sepharose im pH-Bereich von 8,6 bis 9,0; und
das Kationenaustauschchromatographie-Sorptionsmittel ausgewählt ist aus der Gruppe, bestehend aus S-Sepharose, SP-Sepharose und CM-Sepharose, vorzugsweise SP-Sepharose oder CM-Sepharose im pH-Bereich von 5,2 bis 5,6.

17. Fusionsprotein nach den Ansprüchen 1 bis 7, erhalten durch das Verfahren nach den Ansprüchen 11 bis 16.

18. Präparat, umfassend das Fusionsprotein nach Anspruch 17 in Form einer stabilen Lösung.

19. Fusionsprotein nach Anspruch 17 oder Präparat nach Anspruch 18 zur Verwendung in der Therapie.

20. Pharmazeutische Zusammensetzung, umfassend eine therapeutisch wirksame Menge Fusionsprotein nach den Ansprüchen 1 bis 8 oder 17 oder Präparat nach Anspruch 18 und pharmazeutisch annehmbare Träger-, Bindemittel- und/oder Hilfsstoffe.

## Revendications

1. Protéine fusionnée, comprenant un interféron alpha 5 (IFNa5), de formule générale (I) ou (II)
IFNa5-X (I)
X-IFNa5 (II)
dans lesquelles le partenaire de fusion X est un autre interféron alpha ou une autre cytokine.

2. Protéine fusionnée selon la revendication 1, dans laquelle IFNa5 est un interféron alpha 5 humain recombinant, et X est choisi dans le groupe, constitué d'IFNa5, INFa2b et l'oncostatine.

3. Protéine fusionnée selon la revendication 1 ou 2, qui est un homodimère IFNa5-IFNa5 ayant la séquence d'acides aminés de SEQ ID No: 1 ou une séquence au moins 95 % identique.

4. Protéine fusionnée selon la revendication 1 ou 2, qui est un hétérodimère IFNa2b-IFNa5 ayant la séquence d'acides aminés de SEQ ID No: 2 ou une séquence au moins 95 % identique.

5. Protéine fusionnée selon la revendication 1 ou 2, qui est un hétérodimère IFNa5-IFNa2b ayant la séquence d'acides aminés de SEQ ID No: 3 ou une séquence au moins 95 % identique.

6. Protéine fusionnée selon la revendication 1 ou 2, qui est un hétérodimère IFNa5-OSM ayant la séquence d'acides aminés de SEQ ID No: 4 ou une séquence au moins 95 % identique.

7. Protéine fusionnée selon la revendication 1 ou 2, qui est un hétérodimère OSM-IFNa5, ladite séquence d'acides aminés étant SEQ ID No: 5 ou une séquence au moins 95 % identique.

8. Protéine fusionnée selon l'une quelconque des revendications 1 à 7, présentant une activité biologique, caractéristique d'IFNa5 ainsi qu'un taux de clairance prolongé.

9. Fragment d'ADN, codant pour une protéine fusionnée telle que définie dans les revendications 1 à 8, dans lequel la séquence codante est choisie dans le groupe constitué de SEQ ID NO: 6, SEQ ID NO: 7, SEQ ID NO: 8, SEQ ID NO: 9, SEQ ID NO: 10 ou est la séquence codant pour la même protéine.

10. Vecteur, comprenant un fragment d'ADN selon la revendication 9, codant pour une protéine fusionnée telle que définie dans les revendications 1 à 8, ledit vecteur étant choisi dans le groupe constitué de pET21b+, pET28a+, pUC57/T et pT7, et est de préférence pET21b+.

11. Procédé de production d'une protéine fusionnée telle que définie dans les revendications 1 à 8 par expression dans une cellule hôte appropriée, comprenant :
a) la fourniture d'une cellule hôte, produisant ladite protéine fusionnée IFNa5 ;
b) la culture de cellules hôtes dans des conditions efficaces pour exprimer ladite protéine fusionnée IFNa5 dans un milieu de fermentation adapté ;
c) l'isolement et la purification de la protéine fusionnée IFNa5 exprimée, **caractérisé en ce que** :
- ledit isolement est l'isolement à partir du mélange obtenu dans l'étape b), comprenant la protéine fusionnée cible sous la forme de corps d'inclusion, par soumission de ceux-ci à une solubilisation et après renaturation par oxydation ; et
- ladite purification est un traitement chromatographique en 3 étapes comprenant :
la soumission dudit mélange comprenant la protéine fusionnée IFNa5 renaturée à une chromatographie par interaction hydrophobe ;
suivie de la soumission de la solution obtenue dans l'étape précédente à une chromatographie d'échange d'anions ;
et en outre la soumission de la solution obtenue dans l'étape précédente à une chromatographie d'échange de cations.

12. Procédé selon la revendication 11, dans lequel la cellule hôte est choisie dans le groupe constitué de E. coli, S. cerevisiae, K. lactis, de préférence E. coli, plus préférablement une souche déficiente en protéase de E. coli, telle que E. coli BL21 et Rosetta gami-2, de manière préférée entre toutes E. coli BL21, et dans lequel un milieu de fermentation adapté est un milieu complexe quelconque contenant un extrait de levure en tant que source d'azote et du glucose en tant que source de carbone, de préférence du milieu M9m.

13. Procédé selon les revendications 11 à 12, dans lequel lesdites conditions efficaces pour exprimer ladite protéine fusionnée IFN comprend l'induction, de préférence avec de l'isopropyl-β-D-thiogalactopyranoside, de préférence IPTG à une concentration de 0,1 mM à 1 mM, de manière préférée entre toutes 0,5 mM, et l'induction est facultativement décalée à un stade de croissance précoce.

14. Procédé selon les revendications 11 à 13, dans lequel ladite solubilisation est effectuée dans une solution chaotropique et dénaturante, comprenant de l'urée 6 à 8 M ou du chlorhydrate de guanidine 4 à 6 M, de préférence du chlorhydrate de guanidine 6 M, facultativement avec du DTT 20 mM.

15. Procédé selon les revendications 11 à 14, dans lequel ladite renaturation oxydante est conduite dans du tampon glycine/NaOH à un pH dans la plage de 7,0 à 9,6 et une paire de glutathion réduit/oxydé GSH/GSSG dans un rapport de 5 à 20:1 avec des additifs tampons, choisis dans le groupe comprenant le chlorure de sodium, le sulfate de sodium et le sulfate d'ammonium.

16. Procédé selon les revendications 11 à 15, dans lequel, dans ladite purification chromatographique en 3 étapes, le sorbant de chromatographie d'interaction hydrophobe est choisi dans le groupe constitué de Butyl-Sepharose, Octyl-Sepharose, Phenyl-Sepharose, de préférence Phenyl-Sepharose dans une plage de pH de 8,2 à 9,2 ;
le sorbant de chromatographie d'échange d'anions est choisi dans le groupe constitué d'ANX Sepharose, Q-Sepharose, DEAE-Sepharose, QAE-Sepharose, de préférence Q-Sepharose ou DEAE-Sepharose dans la plage de pH de 8,6 à 9,0 ; et
le sorbant de chromatographie d'échange de cations est choisi dans le groupe, constitué de S-Sepharose, SP-Sepharose et CM-Sepharose, de préférence SP-Sepharose ou CM-Sepharose dans la plage de pH de 5,2 à 5,6.

17. Protéine fusionnée telle que définie dans les revendications 1 à 7, obtenue par le procédé selon les revendications 11 à 16.

18. Préparation, comprenant la protéine fusionnée selon la revendication 17 sous la forme d'une solution stable.

19. Protéine fusionnée selon la revendication 17 ou préparation selon la revendication 18 pour utilisation en thérapie.

20. Composition pharmaceutique, comprenant une quantité thérapeutiquement efficace de protéine fusionnée selon les revendications 1 à 8 ou 17 ou préparation selon la revendication 18, et un véhicule, un excipient et/ou des substances auxiliaires pharmaceutiquement acceptables.


### SEQUENCE LISTING

<110> UAB Biotechnologines farmacijos centras "BIOTECHPHARMA"
<120> FUSED PROTEINS OF INTERFERON ALPHA 5 WITH ANOTHER CYTOKINE AND A PROCESS FOR PRODUCING THEREOF
<130> 2830
<160> 24
<170> PatentIn version 3.5
<210> 1
   <211> 333
   <212> PRT
   <213> Homo sapiens
<400> 1
   Met Cys Asp Leu Pro Gln Thr His Ser Leu Ser Asn Arg Arg Thr Leu
   1               5                   10                  15
   Met Ile Met Ala Gln Met Gly Arg Ile Ser Pro Phe Ser Cys Leu Lys
               20                  25                  30
   Asp Arg His Asp Phe Gly Phe Pro Gln Glu Glu Phe Asp Gly Asn Gln
           35                  40                  45
   Phe Gln Lys Ala Gln Ala Ile Ser Val Leu His Glu Met Ile Gln Gln
       50                  55                  60
   Thr Phe Asn Leu Phe Ser Thr Lys Asp Ser Ser Ala Thr Trp Asp Glu
   65                  70                  75                  80
   Thr Leu Leu Asp Lys Phe Tyr Thr Glu Leu Tyr Gln Gln Leu Asn Asp
                   85                  90                  95
   Leu Glu Ala Cys Met Met Gln Glu Val Gly Val Glu Asp Thr Pro Leu
               100                 105                 110
   Met Asn Val Asp Ser Ile Leu Thr Val Arg Lys Tyr Phe Gln Arg Ile
           115                 120                 125
   Thr Leu Tyr Leu Thr Glu Lys Lys Tyr Ser Pro Cys Ala Trp Glu Val
       130                 135                 140
   Val Arg Ala Glu Ile Met Arg Ser Phe Ser Leu Ser Ala Asn Leu Gln
   145                 150                 155                 160
   Glu Arg Leu Arg Arg Lys Glu Cys Asp Leu Pro Gln Thr His Ser Leu
                   165                 170                 175
   Ser Asn Arg Arg Thr Leu Met Ile Met Ala Gln Met Gly Arg Ile Ser
               180                 185                 190
   Pro Phe Ser Cys Leu Lys Asp Arg His Asp Phe Gly Phe Pro Gln Glu
           195                 200                 205
   Glu Phe Asp Gly Asn Gln Phe Gln Lys Ala Gln Ala Ile Ser Val Leu
       210                 215                 220
   His Glu Met Ile Gln Gln Thr Phe Asn Leu Phe Ser Thr Lys Asp Ser
   225                 230                 235                 240
   Ser Ala Thr Trp Asp Glu Thr Leu Leu Asp Lys Phe Tyr Thr Glu Leu
                   245                 250                 255
   Tyr Gln Gln Leu Asn Asp Leu Glu Ala Cys Met Met Gln Glu Val Gly
               260                 265                 270
   Val Glu Asp Thr Pro Leu Met Asn Val Asp Ser Ile Leu Thr Val Arg
           275                 280                 285
   Lys Tyr Phe Gln Arg Ile Thr Leu Tyr Leu Thr Glu Lys Lys Tyr Ser
       290                 295                 300
   Pro Cys Ala Trp Glu Val Val Arg Ala Glu Ile Met Arg Ser Phe Ser
   305                 310                 315                 320
   Leu Ser Ala Asn Leu Gln Glu Arg Leu Arg Arg Lys Glu
                   325                 330
<210> 2
   <211> 332
   <212> PRT
   <213> Homo sapiens
<400> 2
   Met Cys Asp Leu Pro Gln Thr His Ser Leu Gly Ser Arg Arg Thr Leu
   1               5                   10                  15
   Met Leu Leu Ala Gln Met Arg Arg Ile Ser Leu Phe Ser Cys Leu Lys
               20                  25                  30
   Asp Arg His Asp Phe Gly Phe Pro Gln Glu Glu Phe Gly Asn Gln Phe
           35                  40                  45
   Gln Lys Ala Glu Thr Ile Pro Val Leu His Glu Met Ile Gln Gln Ile
       50                  55                  60
   Phe Asn Leu Phe Ser Thr Lys Asp Ser Ser Ala Ala Trp Asp Glu Thr
   65                  70                  75                  80
   Leu Leu Asp Lys Phe Tyr Thr Glu Leu Tyr Gln Gln Leu Asn Asp Leu
                   85                  90                  95
   Glu Ala Cys Val Ile Gln Gly Val Gly Val Thr Glu Thr Pro Leu Met
               100                 105                 110
   Lys Glu Asp Ser Ile Leu Ala Val Arg Lys Tyr Phe Gln Arg Ile Thr
           115                 120                 125
   Leu Tyr Leu Lys Glu Lys Lys Tyr Ser Pro Cys Ala Trp Glu Val Val
       130                 135                 140
   Arg Ala Glu Ile Met Arg Ser Phe Ser Leu Ser Thr Asn Leu Gln Glu
   145                 150                 155                 160
   Ser Leu Arg Ser Lys Glu Cys Asp Leu Pro Gln Thr His Ser Leu Ser
                   165                 170                 175
   Asn Arg Arg Thr Leu Met Ile Met Ala Gln Met Gly Arg Ile Ser Pro
               180                 185                 190
   Phe Ser Cys Leu Lys Asp Arg His Asp Phe Gly Phe Pro Gln Glu Glu
           195                 200                 205
   Phe Asp Gly Asn Gln Phe Gln Lys Ala Gln Ala Ile Ser Val Leu His
       210                 215                 220
   Glu Met Ile Gln Gln Thr Phe Asn Leu Phe Ser Thr Lys Asp Ser Ser
   225                 230                 235                 240
   Ala Thr Trp Asp Glu Thr Leu Leu Asp Lys Phe Tyr Thr Glu Leu Tyr
                   245                 250                 255
   Gln Gln Leu Asn Asp Leu Glu Ala Cys Met Met Gln Glu Val Gly Val
               260                 265                 270
   Glu Asp Thr Pro Leu Met Asn Val Asp Ser Ile Leu Thr Val Arg Lys
           275                 280                 285
   Tyr Phe Gln Arg Ile Thr Leu Tyr Leu Thr Glu Lys Lys Tyr Ser Pro
       290                 295                 300
   Cys Ala Trp Glu Val Val Arg Ala Glu Ile Met Arg Ser Phe Ser Leu
   305                 310                 315                 320
   Ser Ala Asn Leu Gln Glu Arg Leu Arg Arg Lys Glu
                   325                 330
<210> 3
   <211> 332
   <212> PRT
   <213> Homo sapiens
<400> 3
   Met Cys Asp Leu Pro Gln Thr His Ser Leu Ser Asn Arg Arg Thr Leu
   1               5                   10                  15
   Met Ile Met Ala Gln Met Gly Arg Ile Ser Pro Phe Ser Cys Leu Lys
               20                  25                  30
   Asp Arg His Asp Phe Gly Phe Pro Gln Glu Glu Phe Asp Gly Asn Gln
           35                  40                  45
   Phe Gln Lys Ala Gln Ala Ile Ser Val Leu His Glu Met Ile Gln Gln
       50                  55                  60
   Thr Phe Asn Leu Phe Ser Thr Lys Asp Ser Ser Ala Thr Trp Asp Glu
   65                  70                  75                  80
   Thr Leu Leu Asp Lys Phe Tyr Thr Glu Leu Tyr Gln Gln Leu Asn Asp
                   85                  90                  95
   Leu Glu Ala Cys Met Met Gln Glu Val Gly Val Glu Asp Thr Pro Leu
               100                 105                 110
   Met Asn Val Asp Ser Ile Leu Thr Val Arg Lys Tyr Phe Gln Arg Ile
           115                 120                 125
   Thr Leu Tyr Leu Thr Glu Lys Lys Tyr Ser Pro Cys Ala Trp Glu Val
       130                 135                 140
   Val Arg Ala Glu Ile Met Arg Ser Phe Ser Leu Ser Ala Asn Leu Gln
   145                 150                 155                 160
   Glu Arg Leu Arg Arg Lys Glu Cys Asp Leu Pro Gln Thr His Ser Leu
                   165                 170                 175
   Gly Ser Arg Arg Thr Leu Met Leu Leu Ala Gln Met Arg Arg Ile Ser
               180                 185                 190
   Leu Phe Ser Cys Leu Lys Asp Arg His Asp Phe Gly Phe Pro Gln Glu
           195                 200                 205
   Glu Phe Gly Asn Gln Phe Gln Lys Ala Glu Thr Ile Pro Val Leu His
       210                 215                 220
   Glu Met Ile Gln Gln Ile Phe Asn Leu Phe Ser Thr Lys Asp Ser Ser
   225                 230                 235                 240
   Ala Ala Trp Asp Glu Thr Leu Leu Asp Lys Phe Tyr Thr Glu Leu Tyr
                   245                 250                 255
   Gln Gln Leu Asn Asp Leu Glu Ala Cys Val Ile Gln Gly Val Gly Val
               260                 265                 270
   Thr Glu Thr Pro Leu Met Lys Glu Asp Ser Ile Leu Ala Val Arg Lys
           275                 280                 285
   Tyr Phe Gln Arg Ile Thr Leu Tyr Leu Lys Glu Lys Lys Tyr Ser Pro
       290                 295                 300
   Cys Ala Trp Glu Val Val Arg Ala Glu Ile Met Arg Ser Phe Ser Leu
   305                 310                 315                 320
   Ser Thr Asn Leu Gln Glu Ser Leu Arg Ser Lys Glu
                   325                 330
<210> 4
   <211> 363
   <212> PRT
   <213> Homo sapiens
<400> 4
<210> 5
   <211> 363
   <212> PRT
   <213> Homo sapiens
<400> 5
   Met Ala Ala Ile Gly Ser Cys Ser Lys Glu Tyr Arg Val Leu Leu Gly
   1               5                   10                  15
   Gln Leu Gln Lys Gln Thr Asp Leu Met Gln Asp Thr Ser Arg Leu Leu
               20                  25                  30
   Asp Pro Tyr Ile Arg Ile Gln Gly Leu Asp Val Pro Lys Leu Arg Glu
           35                  40                  45
   His Cys Arg Glu Arg Pro Gly Ala Phe Pro Ser Glu Glu Thr Leu Arg
       50                  55                  60
   Gly Leu Gly Arg Arg Gly Phe Leu Gln Thr Leu Asn Ala Thr Leu Gly
   65                  70                  75                  80
   Cys Val Leu His Arg Leu Ala Asp Leu Glu Gln Arg Leu Pro Lys Ala
                   85                  90                  95
   Gln Asp Leu Glu Arg Ser Gly Leu Asn Ile Glu Asp Leu Glu Lys Leu
               100                 105                 110
   Gln Met Ala Arg Pro Asn Ile Leu Gly Leu Arg Asn Asn Ile Tyr Cys
           115                 120                 125
   Met Ala Gln Leu Leu Asp Asn Ser Asp Thr Ala Glu Pro Thr Lys Ala
       130                 135                 140
   Gly Arg Gly Ala Ser Gln Pro Pro Thr Pro Thr Pro Ala Ser Asp Ala
   145                 150                 155                 160
   Phe Gln Arg Lys Leu Glu Gly Cys Arg Phe Leu His Gly Tyr His Arg
                   165                 170                 175
   Phe Met His Ser Val Gly Arg Val Phe Ser Lys Trp Gly Glu Ser Pro
               180                 185                 190
   Asn Arg Ser Arg Arg Cys Asp Leu Pro Gln Thr His Ser Leu Ser Asn
               195                 200                 205
   Arg Arg Thr Leu Met Ile Met Ala Gln Met Gly Arg Ile Ser Pro Phe
       210                 215                 220
   Ser Cys Leu Lys Asp Arg His Asp Phe Gly Phe Pro Gln Glu Glu Phe
   225                 230                 235                 240
   Asp Gly Asn Gln Phe Gln Lys Ala Gln Ala Ile Ser Val Leu His Glu
                   245                 250                 255
   Met Ile Gln Gln Thr Phe Asn Leu Phe Ser Thr Lys Asp Ser Ser Ala
               260                 265                 270
   Thr Trp Asp Glu Thr Leu Leu Asp Lys Phe Tyr Thr Glu Leu Tyr Gln
           275                 280                 285
   Gln Leu Asn Asp Leu Glu Ala Cys Met Met Gln Glu Val Gly Val Glu
       290                 295                 300
   Asp Thr Pro Leu Met Asn Val Asp Ser Ile Leu Thr Val Arg Lys Tyr
   305                 310                 315                 320
   Phe Gln Arg Ile Thr Leu Tyr Leu Thr Glu Lys Lys Tyr Ser Pro Cys
                   325                 330                 335
   Ala Trp Glu Val Val Arg Ala Glu Ile Met Arg Ser Phe Ser Leu Ser
               340                 345                 350
   Ala Asn Leu Gln Glu Arg Leu Arg Arg Lys Glu
           355                 360
<210> 6
   <211> 1002
   <212> DNA
   <213> Artificial Sequence
<220>
   <223> Synthetic coding sequence
<400> 6
   atgtgtgatc tgccgcagac ccactccctg tctaaccgtc gtactctgat gatcatggca     60
   cagatgggtc gtatctctcc tttctcctgc ctgaaggaca gacatgactt tggatttcct    120
   caggaggagt ttgatggcaa ccagttccag aaggctcaag ccatctctgt cctccatgag    180
   atgatccagc agaccttcaa tctcttcagc acaaaggact catctgctac ttgggatgag    240
   acacttctag acaaattcta cactgaactt taccagcagc tgaatgacct ggaagcctgt    300
   atgatgcagg aggttggagt ggaagacact cctctgatga atgtggactc tatcctgact    360
   gtgagaaaat actttcaaag aatcactctc tatctgacag agaagaaata cagcccttgt    420
   gcatgggagg ttgtcagagc agaaatcatg agatccttct ctttatcagc aaacttgcaa    480
   gaacgtttac gtcgtaagga atgtgatctg ccgcagaccc actccctgtc taaccgtcgt    540
   actctgatga tcatggcaca gatgggtcgt atctctcctt tctcctgcct gaaggacaga    600
   catgactttg gatttcctca ggaggagttt gatggcaacc agttccagaa ggctcaagcc    660
   atctctgtcc tccatgagat gatccagcag accttcaatc tcttcagcac aaaggactca    720
   tctgctactt gggatgagac acttctagac aaattctaca ctgaacttta ccagcagctg    780
   aatgacctgg aagcctgtat gatgcaggag gttggagtgg aagacactcc tctgatgaat    840
   gtggactcta tcctgactgt gagaaaatac tttcaaagaa tcactctcta tctgacagag    900
   aagaaataca gcccttgtgc atgggaggtt gtcagagcag aaatcatgag atccttctct    960
   ttatcagcaa acttgcaaga acgtttacgt cgtaaggaat aa                      1002
<210> 7
   <211> 999
   <212> DNA
   <213> Artificial Sequence
<220>
   <223> Synthetic coding sequence
<400> 7
   atgtgtgatc tgccgcagac ccactccctg tctaaccgtc gtactctgat gatcatggca 60
   cagatgggtc gtatctctcc tttctcctgc ctgaaggaca gacatgactt tggatttcct 120
   caggaggagt ttgatggcaa ccagttccag aaggctcaag ccatctctgt cctccatgag 180
   atgatccagc agaccttcaa tctcttcagc acaaaggact catctgctac ttgggatgag 240
   acacttctag acaaattcta cactgaactt taccagcagc tgaatgacct ggaagcctgt 300
   atgatgcagg aggttggagt ggaagacact cctctgatga atgtggactc tatcctgact 360
   gtgagaaaat actttcaaag aatcactctc tatctgacag agaagaaata cagcccttgt 420
   gcatgggagg ttgtcagagc agaaatcatg agatccttct ctttatcagc aaacttgcaa 480
   gaacgtttac gtcgtaagga atgtgatctg ccgcagactc actctctggg ttctcgtcgt 540
   actctgatgc tgctggctca gatgcgtcgt atctctcttt tctcttgcct gaaagaccgt 600
   catgacttcg gtttcccgca ggaagagttc ggtaaccagt tccagaaagc tgaaactatc 660
   cctgttctgc atgaaatgat ccagcaaatc ttcaacctgt tctctactaa agactcttct 720
   gctgcttggg acgaaaccct gctggacaaa ttctacactg aactgtacca gcaactcaac 780
   gacctcgagg cttgtgttat tcagggcgtt ggtgttaccg aaactccgct gatgaaagag 840
   gatagcatcc tggctgttcg caagtatttc cagcgtatca ctctttacct gaaggaaaag 900
   aagtatagcc cgtgtgcttg ggaagttgtt cgtgctgaga tcatgcgttc cttctccctg 960
   tctactaacc tgcaggaatc tctgcgttct aaggaatag 999
<210> 8
   <211> 999
   <212> DNA
   <213> Artificial Sequence
<220>
   <223> Synthetic coding sequence
<400> 8
   atgtgtgatc tgccgcagac tcactctctg ggttctcgtc gtactctgat gctgctggct      60
   cagatgcgtc gtatctctct tttctcttgc ctgaaagacc gtcatgactt cggtttcccg     120
   caggaagagt tcggtaacca gttccagaaa gctgaaacta tccctgttct gcatgaaatg     180
   atccagcaaa tcttcaacct gttctctact aaagactctt ctgctgcttg ggacgaaacc     240
   ctgctggaca aattctacac tgaactgtac cagcaactca acgacctcga ggcttgtgtt     300
   attcagggcg ttggtgttac cgaaactccg ctgatgaaag aggatagcat cctggctgtt     360
   cgcaagtatt tccagcgtat cactctttac ctgaaggaaa agaagtatag cccgtgtgct     420
   tgggaagttg ttcgtgctga gatcatgcgt tccttctccc tgtctactaa cctgcaggaa     480
   tctctgcgtt ctaaggaatg tgatctgccg cagacccact ccctgtctaa ccgtcgtact     540
   ctgatgatca tggcacagat gggtcgtatc tctcctttct cctgcctgaa ggacagacat     600
   gactttggat ttcctcagga ggagtttgat ggcaaccagt tccagaaggc tcaagccatc     660
   tctgtcctcc atgagatgat ccagcagacc ttcaatctct tcagcacaaa ggactcatct     720
   gctacttggg atgagacact tctagacaaa ttctacactg aactttacca gcagctgaat     780
   gacctggaag cctgtatgat gcaggaggtt ggagtggaag acactcctct gatgaatgtg     840
   gactctatcc tgactgtgag aaaatacttt caaagaatca ctctctatct gacagagaag     900
   aaatacagcc cttgtgcatg ggaggttgtc agagcagaaa tcatgagatc cttctcttta     960
   tcagcaaact tgcaagaacg tttacgtcgt aaggaataa                            999
<210> 9
   <211> 1092
   <212> DNA
   <213> Artificial Sequence
<220>
   <223> Synthetic coding sequence
<400> 9
   atgtgtgatc tgccgcagac ccactccctg tctaaccgtc gtactctgat gatcatggca      60
   cagatgggtc gtatctctcc tttctcctgc ctgaaggaca gacatgactt tggatttcct     120
   caggaggagt ttgatggcaa ccagttccag aaggctcaag ccatctctgt cctccatgag     180
   atgatccagc agaccttcaa tctcttcagc acaaaggact catctgctac ttgggatgag      240
   acacttctag acaaattcta cactgaactt taccagcagc tgaatgacct ggaagcctgt      300
   atgatgcagg aggttggagt ggaagacact cctctgatga atgtggactc tatcctgact      360
   gtgagaaaat actttcaaag aatcactctc tatctgacag agaagaaata cagcccttgt      420
   gcatgggagg ttgtcagagc agaaatcatg agatccttct ctttatcagc aaacttgcaa      480
   gaacgtttac gtcgtaagga agcggctata ggcagctgct cgaaagagta ccgcgtgctc      540
   cttggccagc tccagaagca gacagatctc atgcaggaca ccagcagact cctggacccc      600
   tatatacgta tccaaggcct ggatgttcct aaactgagag agcactgcag ggagcgcccc      660
   ggggccttcc ccagtgagga gaccctgagg gggctgggca ggcggggctt cctgcagacc      720
   ctcaatgcca cactgggctg cgtcctgcac agactggccg acttagagca gcgcctcccc      780
   aaggcccagg atttggagag gtctgggctg aacatcgagg acttggagaa gctgcagatg      840
   gcgaggccga acatcctcgg gctcaggaac aacatctact gcatggccca gctgctggac      900
   aactcagaca cggctgagcc cacgaaggct ggccgggggg cctctcagcc gcccaccccc      960
   acccctgcct cggatgcttt tcagcgcaag ctggagggct gcaggttcct gcatggctac     1020
   catcgcttca tgcactcagt ggggcgggtc ttcagcaagt ggggggagag cccgaaccgg     1080
   agccggagat aa                                                          1092
<210> 10
   <211> 1092
   <212> DNA
   <213> Artificial Sequence
<220>
   <223> Synthetic coding sequence
<400> 10
   atggcggcta taggcagctg ctcgaaagag taccgcgtgc tccttggcca gctccagaag 60
   cagacagatc tcatgcagga caccagcaga ctcctggacc cctatatacg tatccaaggc 120
   ctggatgttc ctaaactgag agagcactgc agggagcgcc ccggggcctt ccccagtgag 180
   gagaccctga gggggctggg caggcggggc ttcctgcaga ccctcaatgc cacactgggc 240
   tgcgtcctgc acagactggc cgacttagag cagcgcctcc ccaaggccca ggatttggag 300
   aggtctgggc tgaacatcga ggacttggag aagctgcaga tggcgaggcc gaacatcctc 360
   gggctcagga acaacatcta ctgcatggcc cagctgctgg acaactcaga cacggctgag 420
   cccacgaagg ctggccgggg ggcctctcag ccgcccaccc ccacccctgc ctcggatgct 480
   tttcagcgca agctggaggg ctgcaggttc ctgcatggct accatcgctt catgcactca 540
   gtggggcggg tcttcagcaa gtggggggag agcccgaacc ggagccggag atgtgatctg 600
   ccgcagaccc actccctgtc taaccgtcgt actctgatga tcatggcaca gatgggtcgt 660
   atctctcctt tctcctgcct gaaggacaga catgactttg gatttcctca ggaggagttt 720
   gatggcaacc agttccagaa ggctcaagcc atctctgtcc tccatgagat gatccagcag 780
   accttcaatc tcttcagcac aaaggactca tctgctactt gggatgagac acttctagac 840
   aaattctaca ctgaacttta ccagcagctg aatgacctgg aagcctgtat gatgcaggag 900
   gttggagtgg aagacactcc tctgatgaat gtggactcta tcctgactgt gagaaaatac 960
   tttcaaagaa tcactctcta tctgacagag aagaaataca gcccttgtgc atgggaggtt 1020
   gtcagagcag aaatcatgag atccttctct ttatcagcaa acttgcaaga acgtttacgt 1080
   cgtaaggaat aa 1092
<210> 11
   <211> 27
   <212> DNA
   <213> Artificial Sequence
<220>
   <223> Synthetic primer
<400> 11
   attatcatat gtgtgatctg ccgcaga 27
<210> 12
   <211> 25
   <212> DNA
   <213> Artificial Sequence
<220>
   <223> Synthetic primer
<400> 12
   ttccttacga cgtaaacgtt cttgc 25
<210> 13
   <211> 41
   <212> DNA
   <213> Artificial Sequence
<220>
   <223> Synthetic primer
<400> 13
   ttccttacga cgtaaacgtt cttgctgtga tctgccgcag a 41
<210> 14
   <211> 30
   <212> DNA
   <213> Artificial Sequence
<220>
   <223> Synthetic primer
<400> 14
   gacgtaaacg ttcttgctaa aagcttttaa 30
<210> 15
   <211> 24
   <212> DNA
   <213> Artificial Sequence
<220>
   <223> Synthetic primer
<400> 15
   ttaacatatg tgtgatctgc cgca 24
<210> 16
   <211> 30
   <212> DNA
   <213> Artificial Sequence
<220>
   <223> Synthetic primer
<400> 16
   ttccttagaa cgcagagatt cctgcaggtt 30
<210> 17
   <211> 48
   <212> DNA
   <213> Artificial Sequence
<220>
   <223> Synthetic primer
<400> 17
   tttctgcagg aatctctgcg ttctaaggaa tgtgatctgc cgcagacc 48
<210> 18
   <211> 27
   <212> DNA
   <213> Artificial Sequence
<220>
   <223> Synthetic primer
<400> 18
   tttaagcttc tatctccggc tccggtt 27
<210> 19
   <211> 38
   <212> DNA
   <213> Artificial Sequence
<220>
   <223> Synthetic primer
<400> 19
   ttaacgttta cgtcgtaagg aagcggctat aggcagct 38
<210> 20
   <211> 24
   <212> DNA
   <213> Artificial Sequence
<220>
   <223> Synthetic primer
<400> 20
   aaacatatgg cggctatagg cagc 24
<210> 21
   <211> 18
   <212> DNA
   <213> Artificial Sequence
<220>
   <223> Synthetic primer
<400> 21
   tctccggctc cggttcgg 18
<210> 22
   <211> 39
   <212> DNA
   <213> Artificial Sequence
<220>
   <223> Synthetic primer
<400> 22
   agagcccgaa ccggagccgg agatgtgatc tgccgcaga 39
<210> 23
   <211> 28
   <212> DNA
   <213> Artificial Sequence
<220>
   <223> Synthetic primer
<400> 23
   tacgtcgtaa aggaatgtga tcgccgca 28
<210> 24
   <211> 23
   <212> DNA
   <213> Artificial Sequence
<220>
   <223> Synthetic primer
<400> 24
   aaaaagcttc tattccttag aac 23